(19) Europäisches Patentamt
European Patent Office
Office européen des brevets

(11) EP 1 173 176 B1

(12) EUROPÄISCHE PATENTSCHRIFT

(45) Veröffentlichungstag und Bekanntmachung des Hinweises auf die Patenterteilung:
**26.05.2004 Patentblatt 2004/22**

(21) Anmeldenummer: **00905028.7**

(22) Anmeldetag: **05.02.2000**

(51) Int Cl.[7]: **A61K 31/428**, A61K 31/429, A61K 31/4439, A61K 31/454, A61P 3/04

(86) Internationale Anmeldenummer:
**PCT/EP2000/000925**

(87) Internationale Veröffentlichungsnummer:
**WO 2000/051602 (08.09.2000 Gazette 2000/36)**

(54) **VERWENDUNG VON POLYCYCLISCHEN 2-AMINO-THIAZOL SYSTEMEN ZUR HERSTELLUNG VON MEDIKAMENTEN ZUR PROPHYLAXE ODER BEHANDLUNG VON OBESITAS**

UTILIZATION OF POLYCYCLIC 2-AMINO-THIAZOLE SYSTEMS IN THE PRODUCTION OF MEDICAMENTS FOR PROPHYLAXIS OR TREATMENT OF OBESITY

UTILISATION DE SYSTEMES 2-AMINO-THIAZOLE POLYCYCLIQUES POUR LA preparation DE MEDICAMENTS DESTINES A LA PROPHYLAXIE OU AU TRAITEMENT DE L'OBESITE

(84) Benannte Vertragsstaaten:
**AT BE CH CY DE DK ES FI FR GB GR IE IT LI LU NL PT SE**

(30) Priorität: **26.02.1999 DE 19908537**

(43) Veröffentlichungstag der Anmeldung:
**23.01.2002 Patentblatt 2002/04**

(73) Patentinhaber: **Aventis Pharma Deutschland GmbH**
**65929 Frankfurt am Main (DE)**

(72) Erfinder:
- **JÄHNE, Gerhard**
  **D-65929 Frankfurt (DE)**
- **GEISEN, Karl**
  **D-60318 Frankfurt (DE)**
- **LANG, Hans-Jochen**
  **D-65719 Hofheim (DE)**
- **BICKEL, Martin**
  **D-61348 Bad Homburg (DE)**

(56) Entgegenhaltungen:
**EP-A- 0 230 334**
**EP-A- 0 432 040**
**WO-A-00/04006**
**DE-A- 2 640 358**
**US-A- 3 507 868**

- **GUPTA, RAJIVE ET AL: "Synthesis and antiinflammatory activity of some substituted 2-amino-8H-indeno[1,2-d]thiazoles" INDIAN J. PHARM. SCI. (1991), 53 245-8 , XP000925078 in der Anmeldung erwähnt**
- **RAMALINGAM, K. ET AL: "Synthesis and antimicrobial activity of azasteroid-type compounds and related systems. Effect of hydrophilic and lipophilic groups on activity" J. MED. CHEM. (1977), 20(5), 664-9 , XP000915411**
- **I. KHAZI ET AL.: "Synthesis and biological activity of some 2-amino/arylamino-4H-(1)-benzothiopyrano(4 ,3-d)thiazoles" INDIAN JOURNAL OF HETEROCYCLIC CHEMISTRY, Bd. 4, 1995, Seiten 243-248, XP000933945**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 173 176 B1

## Beschreibung

Die Erfindung betrifft die Verwendung polycyclischer 2-Amino-Thiazol Systeme sowie derer physiologisch verträglichen Salze zur Herstellung von Medikamenten zur Prophylaxe oder Behandlung von Obesitas.

2-Amino-Thiazolsysteme sind in R. Gupta et al., Indian J. Pharm. Sci. 1991, 53, 245-248 als entzündungshemmende Substanzen beschrieben

Der Erfindung lag die Aufgabe zugrunde, Verbindungen zur Verfügung zu stellen, die eine therapeutisch verwertbare anorektische Wirkung entfalten.

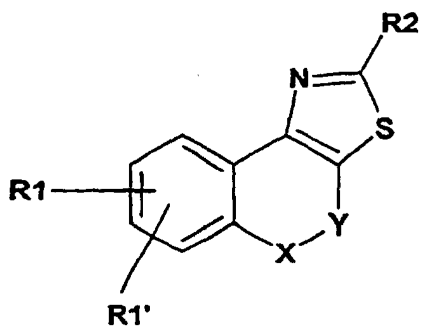

I

Die Erfindung betrifft daher die Verwendung der Verbindungen der Formel I, worin bedeuten

Y eine direkte Bindung, $CH_2$, $CH_2$-$CH_2$;

X $CH_2$, O, NH, NR6, S;

R1, R1' unabhängig voneinander H, F, Cl, Br, J, $CF_3$, CN, $NO_2$, COOH, COO($C_1$-$C_6$)Alkyl, $CONH_2$, CONH($C_1$-$C_6$) Alkyl, CON[($C_1$-$C_6$)Alkyl]$_2$, ($C_1$-$C_6$)-Alkyl, ($C_2$-$C_6$)-Alkenyl, ($C_2$-$C_6$)-Alkinyl, O-($C_1$-$C_6$)-Alkyl, wobei in den Alkyl-, Alkenyl- und Alkinylresten ein, mehrere, oder alle Wasserstoff(e) durch Fluor ersetzt sein können, oder ein Wasserstoff durch OH, OC(O)$CH_3$, OC(O)H, O-$CH_2$-Ph, $NH_2$, NH-CO-$CH_3$ oder N (COO$CH_2$Ph)$_2$ ersetzt sein kann;
$SO_2$-$NH_2$, $SO_2$NH($C_1$-$C_6$)-Alkyl, $SO_2$N[($C_1$-$C_6$)-Alkyl]$_2$ , S-($C_1$-$C_6$)-Alkyl, S-($CH_2$)$_n$-Phenyl, SO-($C_1$-$C_6$) -Alkyl, SO-($CH_2$)$_n$-Phenyl, $SO_2$-($C_1$-$C_6$)-Alkyl, $SO_2$-($CH_2$)$_n$-Phenyl, wobei n = 0 - 6 sein kann und der Phenylrest bis zu zweifach mit F, Cl, Br, OH, $CF_3$, $NO_2$, CN, $OCF_3$, O-($C_1$-$C_6$-Alkyl, ($C_1$-$C_6$)-Alkyl, $NH_2$ substituiert sein kann;
$NH_2$, NH-($C_1$-$C_6$)-Alkyl, N(($C_1$-$C_6$)-Alkyl)$_2$, NH($C_1$-$C_7$)-Acyl, Phenyl, Biphenylyl, O-($CH_2$)$_n$-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenylyl-, Naphthyl-, Pyridyl-, Furanyl- oder Thienylringe jeweils ein bis 3-fach substituiert sein können mit F, Cl, Br. J, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl, $CONH_2$;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;

R2 $NH_2$, NHR3, NR4R5;

R3 ($C_1$-$C_6$)Alkyl, CN, CHO, CO-$NH_2$, CH=NH, C(S)-$NH_2$, C(=NH)-NH-Phenyl, wobei der Phenylring bis zu zweifach substituiert sein kann mit F, Cl, Br, J, OH, $CF_3$, $NO_2$, CN, $OCF_3$, O-($C_1$-$C_6$)-Alkyl, ($C_1$-$C_6$)-Alkyl,

$NH_2$, $NH(C_1-C_6)$-Alkyl, $N((C_1-C_6)$-Alkyl$)_2$, $SO_2-CH_3$, COOH, COO-$(C_1-C_6)$-Alkyl, $CONH_2$;
Phenyl, $CH_2$-Phenyl, wobei der Phenylring ein bis 3-fach substituiert sein kann mit F, Cl, Br, J, OH, $NO_2$, CN, $OCF_3$, O-$(C_2-C_6)$-Alkyl, $(C_2-C_6)$-Alkyl, $NH_2$, $NH(C_1-C_6)$-Alkyl, $N((C_1-C_6)$-Alkyl$)_2$, $SO_2-CH_3$, COOH, COO-$(C_1-C_6)$-Alkyl, $CONH_2$;
Biphenylyl, Naphth-1- oder -2-yl, Pyrid-4-yl, Furan-2- oder -3-yl, Thien-2- oder -3-yl; Tetrazol-5-yl, wobei die Biphenylyl-, Naphthyl-, Pyridyl-, Furanyl- oder Thienylringe jeweils ein bis 2-fach substituiert sein können mit F, Cl, Br, J, OH, $CF_3$, $NO_2$, CN, $OCF_3$, O-$(C_1-C_6)$-Alkyl, $(C_1-C_6)$-Alkyl, $NH_2$, $NH(C_1-C_6)$-Alkyl, $N((C_1-C_6)$-Aikyl$)_2$, $SO_2-CH_3$, COOH, COO-$(C_1-C_6)$-Alkyl, $CONH_2$;
$CH_2$-Phenyl, $CH_2$-Pyrid-2-yl oder $CH_2$-Pyrid-4-yl, wobei wobei der Phenyl- oder Pyridylring ein bis 2-fach substituiert sein kann mit F, Cl, Br, J, OH, $CF_3$, $NO_2$, CN, $OCF_3$, O-$(C_1-C_6)$-Alkyl, $(C_1-C_6)$-Alkyl, $NH_2$, $NH(C_1-C_6)$-Alkyl, $N((C_1-C_6)$-Alkyl$)_2$, $SO_2-CH_3$, COOH, COO-$(C_1-C_6)$-Alkyl, $CONH_2$;

R4 $C_1-C_6$-Alkyl, $C_3-C_6$-Cycloalkyl, $C_2-C_6$-Alkenyl, $C_3-C_6$-Alkinyl, Phenyl, $CH_2$-Phenyl, wobei der Phenylring ein bis 2-fach substituiert sein kann mit F, Cl, Br, J, OH, $CF_3$, $NO_2$, CN, $OCF_3$, O-$(C_1-C_6)$-Alkyl, $(C_1-C_6)$-Alkyl, $NH_2$, $NH(C_1-C_6)$-Alkyl, $N((C_1-C_6)$-Alkyl$)_2$, $SO_2-CH_3$, COOH, COO-$(C_1-C_6)$-Alkyl, $CONH_2$;

R5 H, $C_1-C_6$-Alkyl, $C_3-C_6$-Cycloalkyl, $C_2-C_6$-Alkenyl, $C_3-C_6$-Alkinyl, Phenyl, $CH_2$-Phenyl, wobei der Phenylring ein bis 2-fach substituiert sein kann mit F, Cl, Br, J, OH, $CF_3$, $NO_2$, CN, $OCF_3$, O-$(C_1-C_6)$-Alkyl, $(C_1-C_6)$-Alkyl, $NH_2$, $NH(C_1-C_6)$-Alkyl, $N((C_1-C_6)$-Alkyl$)_2$, $SO_2-CH_3$, COOH, COO-$(C_1-C_6)$-Alkyl, $CONH_2$; oder

R4 und R5 bilden gemeinsam eine der Gruppen $CH_2-CH_2-CH_2-CH_2-Ch_2$, $CH_2-CH_2-N(CH_3)-CH_2-CH_2$, $CH_2-CH_2-N(CH_2$-Phenyl$)-CH_2-CH_2$, $-CH_2-CH_2-O-CH_2-CH_2$, $CH_2-CH_2-CH_2-CH_2$;

R6 $(C_1-C_6)$Alkyl, Acyl, (C=O)-$(C_1-C_6)$Alkyl, (C=O)-$(C_3-C_6)$Cycloalkyl, Phenyl, Naphthyl, Pyridyl, -$SO_2$-Phenyl, -$SO_2$Naphthyl;

sowie derer physiologisch verträglichen Salze zur Herstellung eines Medikaments zur Prophylaxe oder Behandlung von Obesitas.

Bevorzugt ist die Verwendung der Verbindungen der Formel I, in denen ein oder mehrere Rest(e) die folgende Bedeutung hat bzw. haben:

Y eine direkte Bindung, $CH_2$, $CH_2-CH_2$;

X $CH_2O$;

R1 F, Cl, Br, J, $CF_3$, CN, $NO_2$, COOH, COO$(C_1-C_6)$Alkyl, $CONH_2$, CONH$(C_1-C_6)$Alkyl, CON[$(C_1-C_6)$Alkyl$]_2$, $(C_1-C_6)$-Alkyl, $(C_2-C_6)$-Alkenyl, $(C_2-C_6)$-Alkinyl, O-$(C_1-C_6)$-Alkyl, wobei in den Alkyl-, Alkenyl- und Alkinylresten ein, mehrere, oder alle Wasserstoff(e) durch Fluor ersetzt sein können, oder ein Wasserstoff durch OH, $OC(O)CH_3$, OC(O)H, O-$CH_2$-Ph, $NH_2$, NH-CO-$CH_3$ oder $N(COOCH_2Ph)_2$ ersetzt sein kann;
$SO_2-NH_2$, $SO_2NH(C_1-C_6)$-Alkyl, $SO_2N[(C_1-C_6)$-Alkyl$]_2$, S-$(C_1-C_6)$-Alkyl, S-$(CH_2)_n$-Phenyl, SO-$(C_1-C_6)$-Alkyl, SO-$(CH_2)_n$-Phenyl, $SO_2$-$(C_1-C_6)$-Alkyl, $SO_2$-$(CH_2)_n$-Phenyl, wobei n = 0 - 6 sein kann und der Phenylrest bis zu zweifach mit F, Cl, Br, OH, $CF_3$, $NO_2$, CN, $OCF_3$, O-$(C_1-C_6)$-Alkyl, $(C_1-C_6)$-ALkyl, $NH_2$ substituiert sein kann;
Phenyl, -O-Phenyl, wobei der Phenylrest bis zu zweifach substituiert sein kann mit F, Cl, Br, J, OH, $CF_3$, $NO_2$, CN, $OCF_3$, O-$(C_1-C_6)$-Alkyl, $(C_1-C_6)$-Alkyl, $NH_2$, $NH(C_1-C_6)$-Alkyl, $N((C_1-C_6)$-Alkyl$)_2$, $SO_2-CH_3$, COOH, COO-$(C_1-C_6)$-Alkyl, $CONH_2$;

R1' H, F, Cl, Br, J, $CF_3$, CN, $NO_2$, COOH, COO$(C_1-C_6)$Alkyl, $CONH_2$, CONH$(C_1-C_6)$Alkyl, CON[$(C_1-C_6)$Alkyl$]_2$, $(C_1-C_6)$-Alkyl, $(C_2-C_6)$-Alkenyl, $(C_2-C_6)$-Alkinyl, O-$(C_1-C_6)$-Alkyl, wobei in den Alkyl-, Alkenyl- und Alkinylresten ein, mehrere, oder alle Wasserstoff(e) durch Fluor ersetzt sein können, oder ein Wasserstoff durch OH, $OC(O)CH_3$, OC(O)H, O-$CH_2$-Ph, $NH_2$, NH-CO-$CH_3$ oder $N(COOCH_2Ph)_2$ ersetzt sein kann;
$SO_2-NH_2$, $SO_2NH(C_1-C_6)$-Alkyl, $SO_2N[(C_1-C_6$-Alkyl$]_2$, S-$(C_1-C_6)$-Alkyl, S-$(CH_2)_n$-Phenyl, SO-$(C_1-C_6)$-Alkyl, SO-$(CH_2)_n$-Phenyl, $SO_2$-$(C_1-C_6)$-Alkyl, $SO_2$-$(CH_2)_n$-Phenyl, wobei n = 0 - 6 sein kann und der Phenylrest bis zu zweifach mit F, Cl, Br, OH, $CF_3$, $NO_2$, CN, $OCF_3$, O-$(C_1-C_6)$-Alkyl, $(C_1-C_6)$-Alkyl, $NH_2$ substituiert sein kann;
Phenyl, -O-Phenyl, wobei der Phenylrest bis zu zweifach substituiert sein kann mit F, Cl, Br, J, OH, $CF_3$,

$NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl, $CONH_2$;

R2 — $NH_2$, NHR3, NR4R5;

R3 — ($C_1$-$C_6$)Alkyl, CN, CHO, CO-$NH_2$, CH=NH, C(S)-$NH_2$, C(=NH)-NH-Phenyl, wobei der Phenylring bis zu zweifach substituiert sein kann mit F, Cl, Br, J, OH, $CF_3$, $NO_2$, CN, $OCF_3$, O-($C_1$-$C_6$-Alkyl, ($C_1$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl, $CONH_2$;
Phenyl, $CH_2$-Phenyl, wobei der Phenylring ein bis 3-fach substituiert sein kann mit F, Cl, Br, J, OH, $NO_2$, CN, $OCF_3$, O-($C_2$-$C_6$)-Alkyl, ($C_2$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl, $CONH_2$;

R4 — $C_1$-$C_6$-Alkyl, $C_3$-$C_6$-Cycloalkyl, $C_2$-$C_6$-Alkenyl, $C_3$-$C_6$-Alkinyl, Phenyl, $CH_2$-Phenyl, wobei der Phenylring ein bis 2-fach substituiert sein kann mit F,Cl, Br, J, OH, $CF_3$, $NO_2$, CN, $OCF_3$, O-($C_1$-$C_6$)-Alkyl, ($C_1$-$C_6$) -Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl, $CONH_2$;

R5 — $C_1$-$C_6$-Alkyl, $C_3$-$C_6$-Cycloalkyl, $C_2$-$C_6$-Alkenyl, $C_3$-$C_6$-Alkinyl, Phenyl, $CH_2$-Phenyl, wobei der Phenylring ein bis 2-fach substituiert sein kann mit F, Cl, Br, J, OH, $CF_3$, $NO_2$, CN, $OCF_3$, O-($C_1$-$C_6$)-Alkyl, ($C_1$-$C_6$) -Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl, $CONH_2$; oder

R4 und R5 — bilden gemeinsam eine der Gruppen $CH_2$-$CH_2$-$CH_2$-$CH_2$-$CH_2$, $CH_2$-$CH_2$-N($CH_2$-Pheny))-$CH_2$-$CH_2$, $CH_2$-$CH_2$-O-$CH_2$-$CH_2$, $CH_2$-$CH_2$-$CH_2$-$CH_2$;

sowie derer physiologisch verträglichen Salze zur Herstellung eines Medikaments zur Prophylaxe oder Behandlung von Obesitas.

Besonders bevorzugt ist die Verwendung der Verbindungen der Formel I, in denen ein oder mehrere Rest(e) die folgende Bedeutung hat bzw. haben:

Y — eine direkte Bindung;

X — $CH_2$;

R1 — F, Cl, Br, $CF_3$, CN, ($C_1$-$C_6$)-Alkyl, O-($C_1$-$C_6$-Alkyl, wobei in den Alkylresten ein, mehrere, oder alle Wasserstoff(e) durch Fluor ersetzt sein können;
$SO_2$-$NH_2$, $SO_2$NH($C_1$-$C_6$)-Alkyl, $SO_2$N[($C_1$-$C_6$)-Alkyl]$_2$, $SO_2$-($C_1$-$C_6$)-Alkyl, $SO_2$-($CH_2$)$_n$-Phenyl, wobei n = 0 - 6 sein kann und der Phenylrest bis zu zweifach mit F, Cl, Br, OH, $CF_3$, $NO_2$, CN, $OCF_3$, O-($C_1$-$C_6$) -Alkyl, ($C_1$-$C_6$)-Alkyl, $NH_2$ substituiert sein kann;
Phenyl, -O-Phenyl, wobei der Phenylrest bis zu zweifach substituiert sein kann mit F, Cl, Br, OH, $CF_3$, $NO_2$, CN, $OCF_3$, O-($C_1$-$C_6$)-Alkyl, ($C_1$-$C_6$)-Alkyl:
Pyridyl;

R1' — H, F, Cl, Br, $CF_3$, CN, ($C_1$-$C_6$)-Alkyl, O-($C_1$-$C_6$)-Alkyl, wobei in den Alkylresten ein, mehrere, oder alle Wasserstoff(e) durch Fluor ersetzt sein können;
$SO_2NH_2$, $SO_2$NH($C_1$-$C_6$)-Alkyl, $SO_2$N[($C_1$-$C_6$)-Alkyl]$_2$, $SO_2$-($C_1$-$C_6$)-Alkyl, $SO_2$-($CH_2$)$_n$-Phenyl, wobei n = 0 - 6 sein kann und der Phenylrest bis zu zweifach mit F, Cl, Br, OH, $CF_3$, $NO_2$, CN, $OCF_3$, O-($C_1$-$C_6$) -Alkyl, ($C_1$-$C_6$)-Alkyl, $NH_2$ substituiert sein kann;
Phenyl, wobei der Phenylrest bis zu zweifach substituiert sein kann mit F, Cl, Br, OH, $CF_3$, $NO_2$, CN, $OCF_3$, O-($C_1$-$C_6$)-Alkyl, ($C_1$-$C_6$)-Alkyl; Pyridyl;

R2 — $NH_2$, NHR3, NR4R5;

R3 — ($C_1$-$C_6$)Alkyl, CN, CHO, CO-$NH_2$, CH=NH, C(S)-$NH_2$, C(=NH)-NH-Phenyl, wobei der Phenylring bis zu zweifach substituiert sein kann mit F, Cl, Br, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)Alkyl$_2$;

R4 — $C_1$-$C_6$-Alkyl;

R5 — $C_1$-$C_6$-Alkyl; oder

R4 und R5 bilden gemeinsam eine $CH_2$-$CH_2$-$CH_2$-$CH_2$-$CH_2$-Gruppe;

sowie deren physiologisch verträgliche Salze zur Herstellung eines Medikaments zur Prophylaxe oder Behandlung von Obesitas.

Die Erfindung bezieht sich auf die Verwendung der Verbindungen der Formel 1, in Form ihrer Racemate, racemischen Mischungen und reinen Enantiomere sowie auf ihre Diastereomere und Mischungen davon.
Die Alkyl-, Alkenyl- und Alkinylreste in den Substituenten R1, R1', R2, R3, R4, R5 und R6 können sowohl geradkettig wie verzweigt sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der Verbindungen der Formel I sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure. Benzolsulfon-, Benzoe-, Zitronen-, Ethansulfon-, Fumar-, Glucon-, Glykol-, Isäthion-, Milch-, Lactobion-, Malein-, Apfel-, Methansulfon-, Bernstein-, p-Toluolsulfon-, Wein- und Trifluoressigsäure. Für medizinische Zwecke wird in besonders bevorzugter Weise das Chlorid verwendet. Geeignete pharmazeutisch verträgliche basische Salze sind Ammoniumsalze, Alkalimetallsalze (wie Natriumund Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die Verbindungen der Formel I können auch in verschiedenen polymorphen Formen vorliegen, z.B. als amorphe und kristalline polymorphe Formen. Alle polymorphen Formen der Verbindungen der Formel I gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht des vom Salz abgeleiteten Benzothiazepin-Ions. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (1). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung. können in separaten Einheiten vorliegen, wie zum Beispiel Kapseln, Oblatenkapsein, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen

können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete Wirkstoff-Konzentration beträgt ca. 1 % bis 35%, vorzugsweise ca. 3% bis 15%. Als eine besondere Möglichkeit kann der Wirkstoff wie beispielsweise in Pharmaceutical Research, 2(6): 318 (1986) beschrieben, durch Elektrotransport oder lontophorese freigesetzt werden.

Die folgenden Zubereitungen dienen zur Erläuterung der Erfindung ohne diese jedoch einzuschränken.

Beispiel A

Gelatineweichkapseln, enthaltend 100 mg Wirkstoff pro Kapsel:

| | pro Kapsel |
|---|---|
| Wirkstoff | 100 mg |
| aus Kokosfett fraktioniertes Triglycerid-Gemisch | 400 mg |
| Kapselinhalt | 500 mg |

Beispiel B

Emulsion, enthaltend 60 mg Wirkstoff pro 5 ml:

| | pro 100 ml Emulsion |
|---|---|
| Wirkstoff | 1,2 g |

(fortgesetzt)

| | pro 100 ml Emulsion |
|---|---|
| Neutralöl | q.s. |
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

Beispiel C

Rektale Arzneiform, enthaltend 40 mg Werkstoff pro Suppositorium:

| | pro Suppositorium |
|---|---|
| Werkstoff | 40 mg |
| Suppositoriengrundmasse | ad 2 g |

Beispiel D

Tabletten, enthaltend 40 mg Wirkstoff pro Tablette:

| | pro Tablette |
|---|---|
| Lactose | 600 mg |
| Maisstärke | 300 mg |
| lösliche Stärke | 20 mg |
| Magnesiumstearat | 40 mg |
| | 1000 mg |

**Beispiel E**

Dragee, enthaltend 50 mg Wirkstoff pro Dragee:

| | pro Dragee |
|---|---|
| Wirkstoff | 50 mg |
| Maisstärke | 100 mg |
| Lactose | 60 mg |
| sec. Calciumphosphat | 30 mg |
| lösliche Stärke | 5 mg |
| Magnesiumstearat | 10 mg |
| kolloidale Kieselsäure | 5 mg |
| | 260 mg |

Beispiel F

Für die Herstellung des Inhalts von Hartgelatinekapseln eignen sich die folgenden Rezepturen:

| | | |
|---|---|---|
| a) | Wirkstoff | 104mg |
| | Maisstärke, | 300 mg |
| | | 400 mg |

(fortgesetzt)

| | | |
|---|---|---|
| b) | Wirkstoff | 140 mg |
| | Milchzucker | 180 mg |
| | Maisstärke | 180 mg |
| | | 500 mg |

Beispiel G

Tropfen können nach folgender Rezeptur hergestellt werden (100 mg Wirkstoff in 1 ml = 20 Tropfen):

| | |
|---|---|
| Wirkstoff | 10 g |
| Benzoesäuremethylester | 0,07 g |
| Benzoesäureethylester | 0,03 g |
| Ethanol 96 %ig | 5 ml |
| entimineralisiertes Wasser | ad 100 ml |

Gegenstand der Erfindung ist weiterhin ein Verfahren zur Herstellung der Verbindungen der allgemeinen Formel I, dadurch gekennzeichnet, daß man Verbindungen der allgemeinen Formel I gemäß folgendem Reaktionsschema darstellt:

## Reaktionsschema 1:

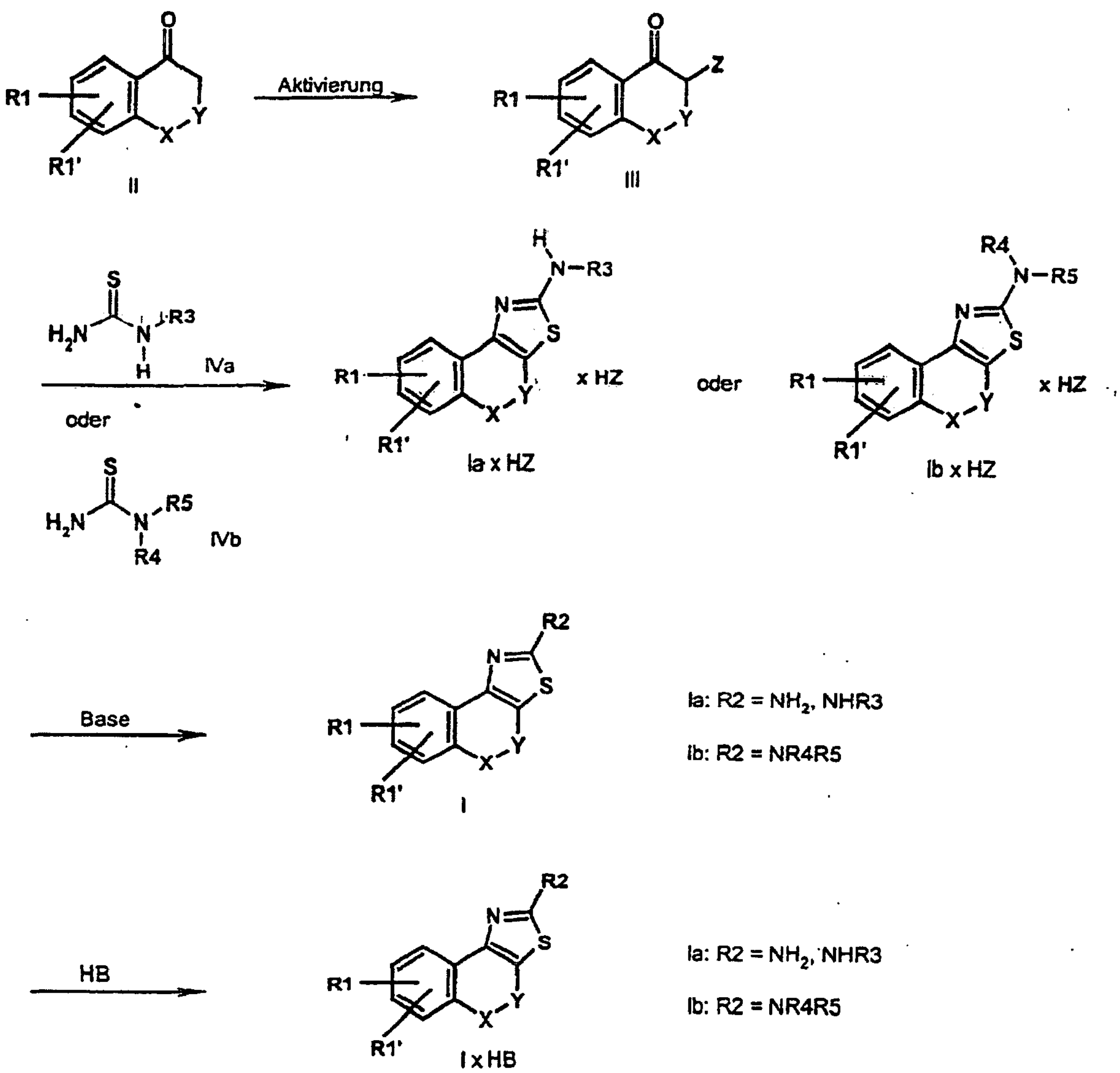


Bicyclische Ketone der allgemeinen Formel II, worin R1, R1', X und Y die angegebenen Bedeutungen besitzen, sind entweder kommerziell erhältlich oder nach literaturbekannten Methoden herstellbar.

Bicyclische Ketone der Formel II, worin R1 oder R1' Arylreste darstellen, können durch Pd(0)-katalysierte Addition von Boronsäureestem an Verbindungen der Formel II, worin R1 und/oder R1' Brom, Jod oder Trifluormethylsulfonyloxy darstellen, gewonnen werden (z.B.: N. Miyaura und A. Suzuki, Chem. Rev. 95, 2457-83 (1995) oder T. Ohe N. Miyaura und A. Suzuki, J. Org. Chem. 58, 2201-08 (1993)).

Bicyclische Ketone der allgemeinen Formel II, worin R1 und/oder R1' Alkinylreste oder Alkenylreste darstellen, können z.B. mit Methoden wie sie bei K. Sonagashira et al., Tetrahedron Lett. 4467 (1975) und S. Takahashi et al., Synthesis 627 (1980) (palladiumkatalysierte Umsetzung von z. B. Trimethylsilyiacetylen oder Alkinen) oder bei E Negishi et al J Org. Chem. 62, 8957-60 (1997) (Alkinylzinkbromide) oder bei A. Hassner et al., J. Org: Chem. 49, 2546 (1984) (Trialkylzinnalkine, Trialkylzinnvinyl- oder allylverbindungen, 1-Alkenylborverbindungen oder Vinylverbindungen) beschrieben sind, hergestellt werden.

Die Aktivierung der bicyclischen Ketone der allgemeinen Formel II erfolgt am einfachsten durch Umsetzung mit Brom zum alpha-Bromketon der allgemeinen Formel III (Z = Br). Z in den aktivierten Verbindungen der allgemeinen Formel III kann jedoch auch vorteilhaft Cl, J. O-C(O)-$C_6H_4$-4-$NO_2$, O-$SO_2$-$CH_3$, O-$SO_2$-$CF_3$, O-$SO_2$-$C_6H_4$-4-$CH_3$ oder O-$SO_2$-$C_6H_4$ sein.

Verbindungen der allgemeinen Formel I x HZ werden gewonnen, indem man Thioharnstoffe der allgemeinen Formel IVa oder IVb, worin R2 = $NH_2$, NHR3 oder R2 = NR4R5 ist und die Reste R2, R3, R4 und R5 die angegebenen

Bedeutungen besitzen umsetzt. Dabei wird vorteilhaft so verfahren, daß man die Verbindungen III mit den Thioharnstoffen IVa oder IVb im molaren Verhältnis von 1:1 bis 1:1,5 umsetzt. Die Reaktion wird vorteilhaft in einem inerten Lösemittel, z.B. in polaren organischen Lösemitteln wie Dimethylformamid, Dimethylacetamid, N-Methyl-2-pyrrolidon, Dimethylsulfoxid, Dioxan, Tetrahydrofuran, Acetonitril, Nitromethan oder Diethylenglykoldimethylether durchgeführt. Als besonders vorteilhafte Lösemittel erweisen sich jedoch Essigsäuremethylester und Essigsäureethylester, kurzkettige Alkohole wie Methanol, Ethanol, Propanol, Isopropanol, sowie niedere Dialkylketone, wie z.B. Aceton, Butan-2-on oder Hexan-2-on. Auch Gemische der angeführten Reaktionsmedien können angewandt werden; so wie auch Gemische der aufgeführten Lösemittel mit Solventien, die für sich alleine genommen weniger geeignet sind, verwendet werden können, wie z.B. Gemische aus Methanol mit Benzol, Ethanol mit Toluol, Methanol mit Diethylether oder mit tert.Butylmethylether, Ethanol mit Tetrachlormethan, Aceton mit Chloroform, Dichlormethan oder 1,2-Dichlorethan, wobei das jeweils polarere Lösemittel zweckmäßigerweise im Überschuß verwendet werden soll. Die Reaktionspartner können im jeweiligen Reaktionsmedium suspendiert oder gelöst vorliegen. Grundsätzlich können die Reaktionspartner auch ohne Lösemittel umgesetzt werden, insbesondere dann,wenn das jeweilige Thioamid einen tiefen Schmelzpunkt hat. Die Reaktion verläuft nur wenig exotherm und kann zwischen -10°C und 150°C, bevorzugt zwischen 50°C und 100°C, durchgeführt werden. Als besonders günstig erweist sich in der Regel ein Temperaturbereich zwischen 50°C und 80°C.

Die Reaktionsdauer ist weitgehend von der Reaktionstemperatur abhängig und liegt zwischen 2 Minuten und 3 Tagen bei höheren bzw. niedrigeren Temperaturen. Im günstigen Temperaturbereich liegt die Reaktionsdauer im allgemeinen zwischen 5 Minuten und 48 Stunden.

Die erhaltenen Salze der Verbindungen der allgemeinen Formel la x HZ und lb x HZ lassen sich mit organischen oder anorganischen Basen in die freien basischen Verbindungen der Formel I (la: R2 = $NH_2$, NHR3; lb: R2 = NR4R5) überführen. Die Verbindungen der allgemeinen Formel I lassen sich durch Umsatz mit organischen oder anorganischen Säuren der Formel HB in ihre Säureadditionssalze der allgemeinen Formel I x HB überführen.Als anorganische Säuren HB kommen beispielsweise in Betracht: Halogenwasserstoffsäuren wie Chlorwasserstoffsäure und Bromwasserstoffsäure, sowie Schwefelsäure, Phosphorsäure und Amidosulfonsäure. Als organische Säuren HB seien beispielsweise genannt: Ameisensäure, Essigsäure, Benzoesäure, p-Toluolsulfonsäure, Benzolsulfonsäure, Bemsteinsäure, Fumarsäure, Maleinsäure, Milchsäure, Weinsäure, Zitronensäure, L-Ascorbinsäure, Salizylsäure, lsäthionsäure, Methansulfonsäure, Trifluormethansulfonsäure, 1,2-Benzisothiazol-3(2H)-on, 6-Methyl-1,2,3-oxathiazin-4(3H)-on-2,2-dioxid.

Erfindungsgemäß wurden außer den in den Ausführungsbeispielen beschriebenen Derivaten auch die in den folgenden Tabellen zusammengestellten Verbindungen der allgemeinen Formel I bzw. deren Säureadditionsprodukte erhalten:

Tabelle 1: Beispiele

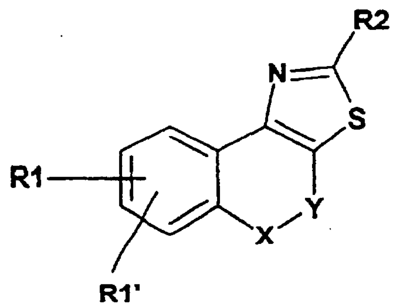

Formel I

| Beispiel | $R_1$; $R_1$' | $R_2$ | Y | X | Salz | Fp. [°C] |
|---|---|---|---|---|---|---|
| 1 | 6-Cl; H | $N(CH_3)_2$ | - | $CH_2$ | HBr | 298 |
| 2 | 6-CN; H | $N(CH_3)_2$ | - | $CH_2$ | HBr | >300 |
| 3 | 6-Cl; H | $NH-C(S)-NH_2$ | - | $CH_2$ | - | 215 |
| 4 | 6-Cl; H | NH-CN | - | $CH_2$ | - | 225 |
| 5 | 6-Cl; H | $N(CH_3)_2$ | - | $CH_2$ | HCl | 219 |
| 6 | 6-Cl; H | NH-CHO | - | $CH_2$ | - | 295 |
| 7 | 6-Cl; H | $NH-C(=NH)-NH-C_6H_5$ | - | $CH_2$ | - | 210 |
| 8 | 6-Cl; H | $NH-(C=NH)-NH-C_6H_4$-4-Cl | - | $CH_2$ | - | 195 |
| 10 | 6-Cl; H | $NH-CO-NH_2$ | | $CH_2$ | HBr | 295 |
| 11 | $5-SO_2-NH_2$; 6-Cl | $NH_2$ | - | $CH_2$ | HBr | 305 |
| 12 | 5-Cl; H | $NH(CH_3)$ | $CH_2$ | O | HCl | 196 |
| 13 | $5-SO_2-CH_3$; H | $NH_2$ | - | $CH_2$ | HCl | >230 |
| 14 | $5-SO_2-CH_3$; H | $NH(CH_3)$ | - | $CH_2$ | HCl | >230 |
| 15 | $5-SO_2-CH_3$; 6 Cl | $NH_2$ | - | $CH_2$ | HCl | >250 |
| 16 | 6-($C_6H_4$-4-$OCH_3$) ; H | $NH(CH_3)$ | - | $CH_2$ | - | 190 |
| 17 | 6-($OC_6H_4$-4-Cl) ; H | $NH(CH_3)$ | - | $CH_2$ | HCl | 241 |

| | | | | | | |
|---|---|---|---|---|---|---|
| 18 | 6-O-$CH_2$-$CF_2$-$CF_3$; H | $NH(CH_3)$ | - | $CH_2$ | HCl | 258 |
| 19 | 6-O-$CH_2$-$CF_3$; H | $NH(CH_3)$ | - | $CH_2$ | HCl | 242 |
| 20 | 6-O-$CH_2$-$CF_3$; H | $NH_2$ | - | $CH_2$ | HCl | 235 |
| 21 | 6-O-$CH_2$-$CF_2$-$CF_3$; H | $NH_2$ | - | $CH_2$ | HCl | 216 |
| 22 | 7-($C_6H_4$-4-$CF_3$) ; H | $NH(CH_3)$ | - | $CH_2$ | - | 221 |
| 23 | 7-($C_6H_4$-4-$CF_3$) ; H | $NH_2$ | - | $CH_2$ | - | 227 |
| 24 | 5-($C_6H_4$-4-Cl) ; H | $N(CH_3)_2$ | - | $CH_2$ | HOAc | 260 |
| 25 | 5-($C_6H_4$-4-$CF_3$) ; H | $NH_2$ | - | $CH_2$ | HBr | 232 |
| 26 | 6-(pyrid-3-yl) ; H | $NH(CH_3)$ | - | $CH_2$ | HCl | 225 |
| 27 | 6-($OC_6H_4$-3-$CH_3$) ; H | $NH_2$ | - | $CH_2$ | HCl | 174 |
| 28 | 6-($OC_6H_4$-3-$CH_3$) ; H | $NH(CH_3)$ | - | $CH_2$ | HCl | 178 |
| 29 | 6-$OC_6H_5$; H | $NH_2$ | - | $CH_2$ | HCl | 148 |
| 30 | 6-O-$CH_2$-$CF_2$-$CF_2$-$CF_3$; H | $NH(CH_3)$ | - | $CH_2$ | HCl | 237 |
| 31 | 5-($C_6H_4$-4-Cl) ; H | $N(CH_3)_2$ | - | $CH_2$ | HBr | 254 |
| 32 | 6-O-$C_6H_4$-4-Cl; H | $NH_2$ | | $CH_2$ | HCl | 216 |
| 33 | 6-Cl; H | piperidin-1-yl | - | $CH_2$ | - | 95 |

Die Verbindungen der Formel I zeichnen sich durch günstige Wirkungen auf den Fettstoffwechsel aus, insbesondere sind sie als Anorektika geeignet. Die Verbindungen können allein oder in Kombination mit weiteren anorektischen Wirkstoffen eingesetzt werden. Solche weiteren anorektischen Wirkstoffe werden z.B. in der Roten Liste, Kapitel 01 unter Abmagerungsmittel/Appetitzügler genannt. Die Verbindungen eignen sich zur Prophylaxe sowie insbesondere zur Behandlung von Obesitas. Die Wirksamkeit der Verbindungen wurde wie folgt getestet:

Biologisches Prüfmodell:

Die Prüfung der anorektischen Wirkung erfolgte an männlichen NMRI Mäusen. Nach 24stündigem Futterentzug wurde über eine Schlundsonde das Testpräparat verabreicht. In Einzelhaltung und bei freiem Zugang zu Trinkwasser wurde den Tieren 30 Minuten nach Präparatgabe Kondensmilch angeboten. Der Kondensmilchverbrauch wurde halbstündlich 7 Stunden lang bestimmt und das Allgemeinbefinden der Tiere beobachtet. Der gemessene Milchverbrauch wurde mit dem unbehandelter Kontrolltieren verglichen.

Tabelle 2: Anorektische Wirkung, gemessen als Reduktion des kumulierten Milchkonsums behandelter im Vergleich zu dem unbehandelter Tiere.

| Verbindung/Beispiel 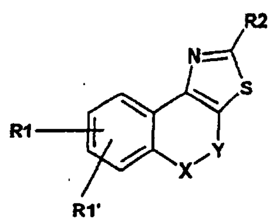 Formel I | Orale Dosis [mg/kg] | Anzahl der Tiere / kumulierten Milchkonsums der behandelten Tiere N / [ml] | Anzahl der Tiere / kumulierten Milchkonsums der unbehandelten Kontrolltiere N / [ml] | Reduktion des kumulierten Milchkonsums in % der Kontrolle |
|---|---|---|---|---|
| Beispiel 1 | 50 | 12 / 1,76 | 12 / 4,05 | 56 |
| Beispiel 2 | 50 | 5 / 0,76 | 5 / 5,14 | 85 |
| Beispiel 6 | 50 | 5 / 0,96 | 5 / 4,20 | 77 |
| Beispiel 18 | 50 | 5 / 0,38 | 5 / 3,84 | 90 |
| Beispiel 19 | 50 | 5 / 0,28 | 5 / 3,82 | 93 |
| Beispiel 20 | 50 | 5 / 0,46 | 5 / 3,82 | 88 |
| Beispiel 26 | 50 | 5 / 0,68 | 5 / 2,94 | 77 |
| Beispiel 27 | 50 | 5 / 0,74 | 5 / 3,54 | 79 |
| Beispiel 30 | 50 | 5 / 0,14 | 5 / 3,58 | 96 |

Die nachfolgend aufgeführten Beispiele dienen zur Erläuterung der Erfindung, ohne diese jedoch einzuschränken. Die angegebenen Zersetzungspunkte sind nicht korrigiert und generell von der Aufheizgeschwindigkeit abhängig.

Ausführungsbeispiel 1:

2-Dimethylamino-8H-indeno[1,2-d]thiazol-6-carbonitril Hydrobromid (Verbindung des Beispiels 02):

a) 1-Oxo-indan-5-carbonitril:

9,5 g 5-Brom-indan-1-on und 4,93 g CuCN werden in 10 ml Dimethylformamid suspendiert und 4 Stunden unter Rückfluß gekocht. Zur abgekühlten, dunkelbraunen, viskosen Suspension wird unter Rühren eine Lösung von 18 g Eisen-III-chlorid in 5 ml konz. Salzsäure mit 30 ml Wasser zugetropft und anschließend für 30 Minuten bei 70°C gerührt. Die Reaktionsmischung wird dreimal mit 50 ml Toluol ausgeschüttelt, und die vereinigten organischen Phasen werden mit 50 ml 2N Salzsäure und 50 ml 2 N Natronlauge ausgeschüttelt und dann mit Wasser neutral gewaschen. Der Toluolextrakt wird über Magnesiumsulfat getrocknet, im Vakuum eingeengt, und der Rückstand wird aus n-Heptan umkristallisiert. Man erhält 1-Oxo-indan-5-carbonitril mit dem Schmelzpunkt 123 - 125°C.

b) 2-Brom-1-oxo-indan-5-carbonitril:
Die Bromierung des 1-Oxo-indan-5-carbonitrils erfolgt mit Brom in Eisessig unter Zufügung einer katalytischen Menge 48%iger HBr-Lösung in Wasser und liefert 2-Brom-1-oxo-indan-5-carbonitril mit dem Schmelzpunkt 115-118°C.

c) 2-Dimethytamino-8H-indeno[1,2-d]thiazol-6-carbonitril Hydrobromid:
236 mg 2-Brom-1-oxo-indan-5-carbonitril werden mit 156 mg N,N-Dimethylthioharnstoff in 10 ml trockenem Aceton 3 h unter Rückfluß erhitzt. Die Reaktionsmischung wird im Vakuum eingeengt; der Rückstand mit wenig Aceton verrührt, abgesaugt, mit Aceton gewaschen und im Vakuum getrocknet. Man erhält 2-Dimethylamino-8H-indeno[1,2-d]thiazol-6-carbonitril Hydrobromid mit dem Schmelzpunkt > 300°C.

Ausführungsbeispiel 2:

N-(6-Chlor 8H-indeno[1,2-d]thiazol-2-yl)-formamid (Verbindung des Beispiels 6):

a) 2-Brom-5-chlor-indan-1-on:
Bromierung von 5-Chlor-indan-1-on in Eisessig mit Brom unter Zugabe katalytischer Mengen 48%iger. HBr-Lösung in Wasser liefert 2-Brom-5-chlor- indan-1-on mit dem Schmelzpunkt: 94-96°C. Als Nebenprodukt wird wenig der entsprechenden Dibromverbindung erhalten.

b) 6-Chlor-8H-indeno[1,2-d]thiazol-2-yl-amin:
10 g 2-Brom-5-chlor-indan-1-on werden bei Raumtemperatur in 300 ml trockenem Methanol gelöst und mit 4.6 g Thioharnstoff versetzt. Nach 5-stündigem Rühren bei Raumtemperatur wird das Lösemittel im Vakuum entfernt, der Rückstand in Aceton verrührt, abgesaugt, mit Aceton gewaschen und im Vakuum getrocknet. Die Suspension des Salzes in Essigsäureethylester wird mit Triethylamin neutralisiert. Die organische Phase wird zweimal mit Wasser und dann zweimal mit ges. Kochsalzlösung gewaschen, über Magnesiumsulfat, getrocknet, filtriert und dann im Vakuum eingeengt. Man erhält 6-Chlor-8H-indeno[1,2-d]thiazol-2-yl-amin, welches ohne weitere Reinigung weiter umgesetzt wird.

c) N-(6-Chlor-8H-indeno[1,2-d]thiazol-2-yl)-formamid:
1.1 g der freien Base werden in einer Mischung aus 19 ml Essigsäureanhydrid und 7.5 ml Ameisensäure 3 h bei 60°C gerührt. Man rührt über Nacht bei Raumtemperatur, danach wird die Reaktionsmischung mit Eiswasser versetzt, kurz gerührt, und der Niederschlag wird abgesaugt und mehrmals mit Wasser gewaschen. N-(6-Chlor-8H-indeno[1,2-d]thiazol-2-yl)-formamid weist nach dem Trocknen im Vakuum einen Schmelzpunkt von 295°C auf.

Ausführungsbeispiel 3:

N-(6-Chlor-8H-indeno[1,2-d]thiazol-2-yl)-N'-phenyl-guanidin (Verbindung des Beispiels 7):

a) (6-Chlor-8H-indeno[1,2-d]thiazol-2-yl)-thiohamstoff Hydrobromid:
10 g 2-Brom-5-chlor-indan-1-on werden bei Raumtemperatur in 400 ml trockenem Ethanol gelöst und mit 11 g Dithiobiuret versetzt. Nach 5 h bei Raumtemperatur wird der gelbe Niederschlag des Hydrobromides des (6-Chlor-8H-indeno[1,2-d]thiazol-2-yl)-thioharnstoffs abgesaugt, mit Ethanol gewaschen und getrocknet und ohne weitere Reinigung in die nächste Stufe eingesetzt.

b) 6-Chlor-8H-indeno[1,2-d]thiazol-2-yl-cyanamid:
3.63 g obiger Verbindung werden unter Rückfluß in 500 ml 2 N Natronlauge gelöst. Zu dieser Lösung werden innerhalb von 20 min. 4.2 g Blei-II.acetat-3-hydrat, gelöst in 50 ml Wasser, langsam zugetropft. Nach einer Stunde wird der Niederschlag (Bleisulfid) heiß abgesaugt. Das Filtrat wird mit Eisessig angesäuert und der gebildete Niederschlag bei 0°C abgesaugt. Weiteres Produkt wird durch Auskochen des Bleisulfidniederschlags mit Methanol gewonnen. Man erhält 6-Chlor 8H-indeno[1,2-d]thiazol-2-ylcyanamid mit dem Schmelzpunkt 225°C.

c) N-(6-Chlor-8H-indeno[1,2-d]thiazol-2-yl)-N'-phenyl-guanidin:
0.26 g 6-Chlor 8H-indeno[1.2-d]thiazol-2-yl-cyanamid werden bei Raumtemperatur in 10 ml Ethanol suspendiert, mit 0.1 ml Anilin versetzt und 6 h zum Rückfluß erwärmt. Die Reaktionsmischung wird im Vakuum eingeengt und der Rückstand zur Reinigung an Kieselgel mit Dichlormethan / Essigsäureethylester 5 / 1 chromatographisch gereinigt. Man erhält N-(6-Chlor-8H-indeno[1,2-d]thiazol-2-yl)-N'-phenyl-guanidin, welches bei 210°C unter Zersetzung schmilzt.

Ausführungsbeispiel 4:

5-Methansulfonyl-8H-indeno[1,2-d]thiazol-2-yl-amin Hydrochlorid (Verbindung des Beispiels 13):

a) 2-Amino-5-methansulfonyl-8,8a-dihydro-indeno[1,2-d]thiazol-3a-ol Hydrochlorid:

2.3 g 2-Brom-6-methansulfonyl-indan-1-on werden in 50 ml Aceton gelöst und unter Rühren mit 0.67 g Thioharnstoff versetzt. Aus der zunächst klaren Lösung kristallisiert nach einigen Minuten das Hydrobromid der ringeschlossenen Verbindung aus. Man rührt 4 h bei Raumtemp. nach, saugt ab, löst den Feststoff in ca. 30 ml Methanol und setzt 1 ml Triethylamin zu. Wiederum setzt nach wenigen Minuten Niederschlagsbildung ein. Nach 15 min gibt man 150 ml Wasser dazu und vervollständigt die Produktbildung durch Nachrühren bei Raumtemp. Der Niederschlag wird abgesaugt, mit Wasser nachgewaschen und an der Luft getrocknet. Durch Lösen in Essigester, Zusatz von etherischer Salzsäure, Absaugen des gebildeten Produkts und Trocknen i. Vak. erhält man das Hydrochlorid des 2-Amino-5-methansulfonyl-8,8a-dihydro-indeno[1,2-d]thiazol-3a-ols mit dem Zersetzungspunkt 241°C.

b) 5-Methansulfonyl-8H-indeno[1,2-d]thiazol-2-yl-amin Hydrochlorid:

1g der unter a) gewonnenen Verbindung wird in 100 ml halbconc. Salzsäure 10 h bei Raumtemp. gerührt, das Produkt abgesaugt und mit kaltem Wasser kurz nachgewaschen. Man erhält 5-Methansulfonyl-8Hindeno[1,2-d] thiazol-2-yl-amin Hydrochlorid vom Schmelzpunkt 230 °C.

Ausführungsbeispiel 5:

6-Chlor-5-methansulfonyl-8H-indeno[1,2-d]thiazol-2-yl-amin Hydrochlorid (Verbindung des Beispiels 15):

Ausgehend von 2-Brom-5-chlor-6-methansulfonyl-indan-1-on gewinnt man in der Art und Weise wie vorgehend beschrieben 6-Chlor-5-methansulfonyl-8H-indeno[1,2-d]thiazol-2-yl-amin Hydrochlorid mit dem Schmelzpunkt >260°C.

Ausführungsbeispiel 6:

Methyl-[6-(2,2,3,3,3-pentafluor-propoxy)-8H-indeno[1,2-d]thiazol-2-yl]-amin Hydrochlorid (Verbindung des Beispiels 18):

a) 5-(2.2,3.3,3-pentafluor-propoxy)-indan-1-on:

6.5 g 5-Fluor-indan-1 -on werden in 50 ml trockenem Dimethylacetamid gelöst, mit 35.6 g wasserfreiem, gemahlenem-Kaliumcarbonat und 12.9 g 2,2,3.3,3-Pentafluorpropanot versetzt und 10h bei 95-100°C gerührt. Danach wird das Lösemittel im Vakuum abdestilliert; der Rückstand wird mit 300 ml Wasser versetzt, und die wässrige Phase wird mehrmals mit Essigsäureethylester extrahiert. Die organische Phase wird mit Wasser gewaschen, über Natriumsulfat getrocknet und im Vakuum eingeengt. Die Reinigung an Kieselgel liefert 5-(2,2,3,3,3-pentafluor-propoxy)-indan-1-on als ein braunes Öl, welches nach einiger Zeit kristallisiert; Schmelzpunkt 52-54°C.

b) 2-Brom-5-(2,2,3,3,3-pentafluor-propoxy)-indan-1-on:

6.9 g 5-(2,2,3,3,3-pentafluor-propoxy)-indan-1-on werden in 100 ml Essigsäureethylester gelöst und tropfenweise mit einer Lösung von 3.9 g Brom in 15 ml Essigsäureethylester versetzt. Die Lösung wird kurz zum Rückfluß erhitzt, bevor der Rest der Bromlösung zugetropft wird. Anschließend wird 2 h bei Raumtemperatur gerührt. Die Reaktionslösung wird im Vakuum eingeengt und liefert 2-Brom-5-(2,2,3,3,3-pentafluorpropoxy)-indan-1-on als Öl, welches ohne weitere Reinigung in die nächste Stufe eingesetzt wird.

c) Methyl-[6-(2,2,3,3,3-pentafluor-propoxy)-8H-indeno[1,2-d]thiazol-2-yl]-amin Hydrochlorid:

1.79 g 2-Brom-5-(2,2,3,3,3-pentafluor propoxy)-indan-1-on werden in 60 ml Essigsäureethylester gelöst und mit einer Suspension aus 450 mg N-Methylthiohamstoff in 20 ml Essigsäureethylester versetzt. Die Reaktionslösung wird 7 h bei Raumtemperatur gerührt; der helle Niederschlag wird abgesaugt und mit Essigsäureethylester

gewaschen und anschließend getrocknet. Das erhaltene Hydrobromid wird in 60 ml Methanol gelöst, mit 1.53 g Triethylamin versetzt und 5 h bei Raumtemperatur gerührt. Die Lösung wird eingeengt; der Rückstand kristallisiert bei Zugabe von Wasser. Die getrocknete freie Base wird in Essigsäureethylester gelöst und mit etherischer HCl-Lösung bis zur sauren Reaktion versetzt. Nach 3 h bei Raumtemperatur werden die gebildeten Kristalle abgesaugt und im Vakuum getrocknet. Zur Herstellung des ungesättigten Systems werden die getrockneten Kristalle in 35 ml Eisessig 2 h zum Rückfluß erhitzt. Das Lösemittel wird im Vakuum abdestilliert und der feste Rückstand wird mit Diisopropylether verrührt, abgesaugt und im Vakuum getrocknet. Man erhält Methyl-[6-(2,2,3.3,3-pentafluor-propoxy)-8H-indeno[1,2-d]thiazol-2-yl]-amin Hydrochlorid mit dem Schmelzpunkt 258°C.

Ausführungsbeispiel 7:

Methyl-(6-pyridin-3-yl-8H-indeno[1,2-d]thiazol-2-yl)-amin Hydrochlorid (Verbindung des Beispiels 26):

a) 5-Pyridin-3-yl-indan-1-on:

13,26 g 3-Brompyridin werden in 160 ml Diethylether gelöst und auf-60°C abgekühlt. Zu dieser Lösung werden während 30 Minuten 52 ml einer 1,6 molaren Lösung von n-Butyllithium in n-Hexan zugetropft. Man läßt die Lösung sich auf -30°C erwärmen und fügt bei dieser Temperatur tropfenweise unter Rühren 9,5 ml Borsäuretrimethylester zu. Das Reaktionsgemisch wird anschließend 3 Stunden unter Rückfluß erhitzt, dann auf 0°C abgekühlt und tropfenweise mit 6,1 ml 1,3-Propandiol versetzt. Diese Mischung wird 30 Minuten bei 0°C gerührt, bevor 5,46 ml Methansulfonsäure zugetropft werden und weitere 30 Minuten gerührt wird. Danach fügt man 20 g Celite zu, erwärmt das Gemisch auf Raumtemperatur, filtriert, engt das Filtrat ein, verrührt den Rückstand in 700 ml Toluol, filtriert erneut und destilliert das Lösemittel im Vakuum ab. 4,1 g des Rückstands (3-[1,3,2]Dioxaborinan-2-yl-pyridin) werden ohne weitere Reinigung zusammen mit 4,22 g 5-Bromindan-1-on und 4,24 g Natriumcarbonat in einer Mischung aus 100 ml Toluol mit 20 ml Ethanol und 20 ml Wasser gelöst. Die Lösung wird mit Argon entgast, und anschließend werden 112 mg Palladium-II-acetat und 262 mg Triphenylphosphin zugegeben. Das Reaktionsgemisch wird 4 Stunden unter Rückfluß gekocht, auf Raumtemperatur abgekühlt und der Ethanolanteil des Gemisches wird im Vakuum abdestilliert. Sodann werden unter Rühren 50 ml einer 0,5 N Natronlauge zugesetzt, die organische Phase abgetrennt und die wässrige Phase mit Toluol ausgeschüttelt. Die vereinigten organischen Phasen werden nacheinander mit Wasser und ges. Kochsalzlösung ausgeschüttelt, über Magnesiumsulfat getrocknet, im Vakuum eingeengt und über Kieselgel mit Essigsäureethylester /n-Heptan 1/1 chromatographisch gereinigt. Man erhält 5-pyridin-3-yl-indan-1-on mit dem Schmelzpunkt 103 -106°C.

b) 2-Chlor-5-pyridin-3-yl-indan-1-on:

3,22 g 5-pyridin-3-yl-indan-1-on werden in 160 ml Dichlormethan gelöst und bei 0°C während 15 Minuten tropfenweise mit einer Lösung von 1,34 ml Sulfurylchlorid in 40 ml Dichlormethan versetzt. Es wird 30 Minuten bei 0°C und dann 60 Minuten bei Raumtemperatur nachgerührt, bevor man langsam 50 ml einer ges. Natriumhydrogencarbonatlösung zufügt. Die organische Phase wird abgetrennt, mit Wasser gewaschen, über Magnesiumsulfat getrocknet, im Vakuum eingeengt und über Kieselgel mit Dichlormethan / Methanol 50/1 chromatographisch gereinigt. Man erhält (neben 2,2-Dichlor-5-pyridin-3-yl-indan-1-on mit dem Schmelzpunkt 109°C) 2-Chlor-5-pyridin-3-yl-indan-1-on mit dem Schmelzpunkt 103-105°C.

c) Methyl-(6-pyridin-3-yl-8H-indeno[1,2-d]thiazol-2-yl)-amin Hydrochlorid:

366 mg 2-Chlor-5-pyridin-3-yl-indan-1-on werden mit 203 mg N-Methylthiohamstoff in 5 ml Methanol gelöst und 7 h zum Rückfluß erhitzt. Die abgekühlte Reaktionsmischung wird mit 20 ml Aceton versetzt, der Niederschlag wird abgesaugt, mit Aceton gewaschen und im Vakuum getrocknet. Man erhält Methyl-(6-pyridin-3-yl-8H-indeno [1,2-d]thiazol-2-yl)-amin Hydrochlorid mit dem Schmelzpunkt 225°C.

Ausführungsbeispiel 8:

6-m-Tolyloxy-8H-indeno[1,2-d]thiazol-2-yl-amin Hydrochlorid (Verbindung des Beispiels 27):

a) 5-m-Tolyloxy-indan-1-on:

5 g 5-Fluor-indan-1-on werden in 50 ml trockenem Dimethylformamid gelöst und mit 18.2 g wasserfreiem, gepulvertem Kaliumcarbonat und 3.57 g m-Kresol versetzt. Die Reaktionsmischung wird 6 h bei 110°C gerührt.

Das Lösemittel wird im Vakuum abdestilliert und der Rückstand wird mit 100 ml Wasser versetzt und 2 h gerührt. Der wässrige Rückstand wird mit Essigsäureethylester extrahiert und der organische Extrakt wird 3 x mit Wasser gewaschen, über Natriumsulfat getrocknet, filtriert und im Vakuum eingeengt. Man erhält 5-m-Tolyloxy indan-1-on als ein braunes Öl, welcher ohne weitere Reinigung weiter umgesetzt wird.

b) 2-Brom-5-m-tolyloxy-indan-1 -on:

Die Bromierung von 5-m-Tolyloxy-indan-1 -on erfolgt analog der Bromierung von 5-(2,2,3,3,3-pentafluor-propoxy)-indan-1-on (Ausführungsbeispiel 6) und liefert 2-Brom-5-m-tolyloxy-indan-1-on als hellbraunes Öl.

c) 6-m-Tolyloxy-8H-indeno[1,2-d]thiazol-2-yl-amin Hydrochlorid:

1.4 g des obigen Bromketons werden in 14 ml Aceton gelöst, mit 340 mg Thioharnstoff in 20 ml Aceton vesetzt und 7 h bei Raumtemperatur gerührt. Die ausgefallenen Kristalle (2-Amino-6-m-tolyloxy-8,8a-dihydro-indeno [1,2-d]thiazol-3a-ol Hydrobromid) werden abgesaugt und im Vakuum getrocknet. Wie im Ausführungsbeispiel 6 beschrieben, wird auch dieses Hydrobromid in die freie Base und weiter in das Hydrochlorid überführt. Das Hydrochlorid des 2-Amino-6-m-tolyloxy-8,8a-dihydro-indeno[1,2-d]thiazol-3a-ols wird in 30 ml Eisessig supendiert und unter Rühren zum Rückfluß erhitzt. Nach 2 h wird die Lösung im Vakuum eingeengt, der Rückstand mit Diisopropylether verrührt, abgesaugt und im Vakuum getrocknet. Man erhält 6-m-Tolyloxy-8H-indeno[1,2-d]thiazol-2-yl-amin Hydrochlorid mit dem Schmelzpunkt 174°C.

## Patentansprüche

**1.** Verwendung der Verbindungen der Formel I,

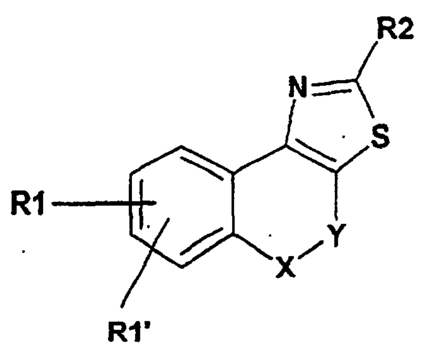

I

worin bedeuten

Y eine direkte Bindung, $CH_2$, $CH_2$-$CH_2$;

X $CH_2$, O, NH, NR6, S;

R1, R1' unabhängig voneinander H, F, Cl, Br, J, $CF_3$, CN, $NO_2$, COOH, COO($C_1$-$C_6$)Alkyl, $CONH_2$, CONH ($C_1$-$C_6$)Alkyl, CON[($C_1$-$C_6$)Alkyl]$_2$, ($C_1$-$C_6$)-Alkyl, ($C_2$-$C_6$)-Alkenyl, ($C_2$-$C_6$)-Alkinyl, O-($C_1$-$C_6$)-Alkyl, wobei in den Alkyl-, Alkenyl- und Alkinylresten ein, mehrere, oder alle Wasserstoff(e) durch Fluor ersetzt sein können, oder ein Wasserstoff durch OH, OC(O)$CH_3$, OC(O)H, O-$CH_2$-Ph, $NH_2$, NH-CO-$CH_3$ oder N($COOCH_2Ph$)$_2$ ersetzt sein kann;
$SO_2$-$NH_2$, $SO_2$NH($C_1$-$C_6$)-Alkyl, $SO_2$N[($C_1$-$C_6$)-Alkyl]$_2$, S-($C_1$-$C_6$)-Alkyl, S-($CH_2$)$_n$-Phenyl, SO-($C_1$-$C_6$)-Alkyl, SO-($CH_2$)$_n$-Phenyl, $SO_2$-($C_1$-$C_6$)-Alkyl, $SO_2$-($CH_2$)$_n$-Phenyl, wobei n = 0 - 6 sein kann und der Phenylrest bis zu zweifach mit F, Cl, Br, OH, $CF_3$, $NO_2$, CN, $OCF_3$, O-($C_1$-$C_6$)-Alkyl, ($C_1$-$C_6$)-Alkyl, $NH_2$ substituiert sein kann;
$NH_2$, NH-($C_1$-$C_6$)-Alkyl, N(($C_1$-$C_6$)-Alkyl)$_2$, NH($C_1$-$C_7$)-Acyl, Phenyl, Biphenylyl, O-($CH_2$)$_n$-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenylyl-, Naphthyl-, Pyridyl-, Furanyl- oder Thienylringe jeweils ein

bis 3-fach substituiert sein können mit F, Cl, Br; J, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl, $CONH_2$; 1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;

R2 $NH_2$, NHR3, NR4R5;

R3 ($C_1$-$C_6$)Alkyl, CN, CHO, CO-$NH_2$, CH=NH, C(S)-$NH_2$, C(=NH)-NH-Phenyl, wobei der Phenylring bis zu zweifach substituiert sein kann mit F, Cl, Br, J, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SO_2$-$CH_3$. COOH, COO-($C_1$-$C_6$)-Alkyl, $CONH_2$;
Phenyl, $CH_2$-Phenyl, wobei der Phenylring ein bis 3-fach substituiert sein kann mit F, Cl, Br, J, OH, $NO_2$, CN, $OCF_3$, O-($C_2$-$C_6$)-Alkyl, ($C_2$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl, $CONH_2$;
Biphenylyl, Naphth-1- oder -2-yl, Pyrid-4-yl, Furan-2- oder -3-yl, Thien-2- oder -3-yl, Tetrazol-5-yl, wobei die Biphenylyl-, Naphthyl-, Pyridyl-, Furanyl- oder Thienylringe jeweils ein bis 2-fach substituiert sein können mit F, Cl, Br, J, OH, $CF_3$, $NO_2$, CN, $OCF_3$, O-($C_1$-$C_6$)-Alkyl, $C_1$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl, $CONH_2$;
$CH_2$-Phenyl, $CH_2$-Pyrid-2-yl oder $CH_2$-Pyrid-4-yl, wobei der Phenyl- oder Pyridylring ein bis 2-fach substituiert sein kann mit F, Cl, Br, J, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl, $CONH_2$;

R4 $C_1$-$C_6$-Alkyl, $C_3$-$C_6$-Cycloalkyl, $C_2$-$C_6$-Alkenyl, $C_3$-$C_6$-Alkinyl, Phenyl, $CH_2$-Phenyl, wobei der Phenylring ein bis 2-fach substituiert sein kann mit F, Cl, Br, J, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl, $CONH_2$;

R5 H, $C_1$-$C_6$-Alkyl, $C_3$-$C_6$-Cycloalkyl, $C_2$-$C_6$-Alkenyl, $C_3$-$C_6$-Alkinyl, Phenyl, $CH_2$-Phenyl, wobei der Phenylring ein bis 2-fach substituiert sein kann mit F, Cl, Br, J, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)Alkyl, N(($C_1$-$C_6$)-Alkyl)$_2$, $SO_2$-$CH_3$, COOH, COO-($C_1$-$C_6$)-Alkyl, $CONH_2$; oder

R4 und R5 bilden gemeinsam eine der Gruppen $CH_2$-$CH_2$-$CH_2$-$CH_2$-$CH_2$, $CH_2$-$CH_2$-N($CH_3$)-$CH_2$-$CH_2$, $CH_2$-$CH_2$-N($CH_2$-Phenyl)-$CH_2$-$CH_2$, $CH_2$-$CH_2$-O-$CH_2$-$CH_2$, $CH_2CH_2$-$CH_2$-$CH_2$;

R6 ($C_1$-$C_6$)Alkyl, Acyl, (C=O)-($C_1$-$C_6$)Alkyl, (C=O)-($C_3$-$C_6$)Cycloalkyl, Phenyl, Naphthyl, Pyridyl, -$SO_2$-Phenyl, -$SO_2$-Naphthyl;

sowie derer physiologisch verträglichen Salze zur Herstellung eines Medikaments zur Prophylaxe oder Behandlung von Obesitas.

**2.** Verwendung der Verbindungen der Formel I, gemäß Anspruch 1, **dadurch gekennzeichnet, daß** darin bedeuten

Y eine direkte Bindung, $CH_2$, $CH_2$-$CH_2$;

X $CH_2O$;

R1 F, Cl, Br, J, $CF_3$, CN, $NO_2$, COOH, COO($C_1$-$C_6$,)Alkyl, $CONH_2$, CONH($C_1$-$C_6$)Alkyl, CON[($C_1$-$C_6$)Alkyl]$_2$, ($C_1$-$C_6$)-Alkyl, ($C_2$-$C_6$)-Alkenyl, ($C_2$-$C_6$)-Alkinyl, O-($C_1$-$C_6$)-Alkyl, wobei in den Alkyl-, Alkenyl- und Alkinylresten ein, mehrere, oder alle Wasserstoff(e) durch Fluor ersetzt sein können, oder ein Wasserstoff durch OH, OC(O)$CH_3$, OC(O)H, O-$CH_2$-Ph, $NH_2$, NH-CO-$CH_3$ oder N($COOCH_2$Ph)$_2$ ersetzt sein kann;
$SO_2$-$NH_2$, $SO_2$NH($C_1$-$C_6$)-Alkyl, $SO_2$N[($C_1$-$C_6$)-Alkyl]$_2$, S-($C_1$-$C_6$)-Alkyl, S-($CH_2$)$_n$-Phenyl, SO-($C_1$-$C_6$)-Alkyl, SO-($CH_2$)$_n$-Phenyl, $SO_2$-(Cl-$C_6$)-Alkyl, $SO_2$-($CH_2$)$_n$-Pheny), wobei n = 0 - 6 sein kann und der Phenylrest bis zu zweifach mit F, Cl, Br, OH, $CF_3$, $NO_2$, CN, $OCF_3$,O-($C_1$-$C_6$)-Alkyl, ($C_1$-$C_6$)-Alkyl, $NH_2$ substituiert sein kann;
Phenyl, -O-Phenyl, wobei der Phenylrest bis zu zweifach substituiert sein kann mit F, Cl, Br, J, OH,

$CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl, $CONH_2$;

R1' H, F, Cl, Br, J, $CF_3$, CN, $NO_2$, COOH, COO($C_1$-$C_6$)Alkyl, $CONH_2$, CONH($C_1$-$C_6$)Alkyl, CON[($C_1$-$C_6$) Alkyl]$_2$, ($C_1$-$C_6$)-Alkyl, ($C_2$-$C_6$)-Alkenyl, ($C_2$-$C_6$)-Alkinyl, O-($C_1$-$C_6$)-Alkyl, wobei in den Alkyl-, Alkenyl- und Alkinylresten ein, mehrere, oder alle Wasserstoff(e) durch Fluor ersetzt sein können, oder ein Wasserstoff durch OH, OC(O)$CH_3$, OC(O)H, O-$CH_2$-Ph, $NH_2$, NH-CO-$CH_3$ oder N(COOC$H_2$Ph)$_2$ ersetzt sein kann;
$SO_2$-$NH_2$, $SO_2$NH($C_1$-$C_6$)-Alkyl, $SO_2$N[($C_1$-$C_6$)-Alkyl]$_2$ , S-($C_1$-$C_6$)-Alkyl, S-($CH_2$)$_n$-Phenyl, SO-($C_1$-$C_6$)-Alkyl, SO-($CH_2$)$_n$-Phenyl, $SO_2$-($C_1$-$C_6$)-Alkyl, $SO_2$-($CH_2$)$_n$-Phenyl, wobei n = 0 - 6 sein kann und der Phenylrest bis zu zweifach mit F, Cl, Br, OH, $CF_3$, $NO_2$, CN, $OCF_3$, O-($C_1$-$C_6$)-Alkyl, ($C_1$-$C_6$)-Alkyl, $NH_2$ substituiert sein kann;
Phenyl, -O-Phenyl, wobei der Phenylrest bis zu zweifach substituiert sein kann mit F, Cl, Br, J, OH, $CF_3$, $NO_2$, CN, $OCF_3$, O-($C_1$-$C_6$)-Alkyl. ($C_1$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl, $CONH_2$:

R2 $NH_2$, NHR3, NR4R5:

R3 ($C_1$-$C_6$)Alkyl, CN, CHO, CO-$NH_2$, CH=NH, C(S)-$NH_2$, C(=NH)-NH-Phenyl, wobei der Phenylring bis zu zweifach substituiert sein kann mit F, Cl, Br, J, OH, $CF_3$, $NO_2$, CN, $OCF_3$, O-($C_1$-$C_6$)-Alkyl, ($C_1$-$C_6$) -Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl, $CONH_2$;
Phenyl, $CH_2$-Phenyl, wobei der Phenylring ein bis 3-fach substituiert sein kann mit F, Cl, Br, J, OH, $NO_2$, CN, $OCF_3$, O-($C_2$-$C_6$)-Alkyl, ($C_2$-$C_6$)-Alkyl, $NH_2$, NH($C_1$-$C_6$-Alkyl, N(($C_1$-$C_6$)-Alkyl)$_2$, $SO_2$-$CH_3$, COOH, COO-($C_1$-$C_6$)-Alkyl, $CONH_2$;

R4 $C_1$-$C_6$-Alkyl, $C_3$-$C_6$-Cycloalkyl, $C_2$-$C_6$-Alkenyl, $C_3$-$C_6$-Alkinyl, Phenyl, $CH_2$-Phenyl, wobei der Phenylring ein bis 2-fach substituiert sein kann mit F, Cl, Br, J, OH, $CF_3$, $NO_2$, CN, $OCF_3$, O-($C_1$-$C_6$) -Alkyl, ($C_1$-$C_6$)-Alkyl, $NH_2$, NH($C_1$-$C_6$)-Alkyl, N(($C_1$-$C_6$)Alkyl, $SO_2$-$CH_3$, COOH, COO-($C_1$-$C_6$)-Alkyl, $CONH_2$;

R5 $C_1$-$C_6$-Alkyl, $C_3$-$C_6$-Cycloalkyl, $C_2$-$C_6$-Alkenyl, $C_3$-$C_6$-Alkinyl, Phenyl, $CH_2$-Phenyl, wobei der Phenylring ein bis 2-fach substituiert sein kann mit F, Cl, Br, J, OH, $CF_3$, $NO_2$, CN, $OCF_3$, O-($C_1$-$C_6$) -Alkyl, ($C_1$-$C_6$)-Alkyl, $NH_2$, NH($C_1$-$C_6$)-Alkyl, N(($C_1$-$C_6$)-Alkyl)$_2$, $SO_2CH_3$, COOH, COO-($C_1$-$C_6$)-Alkyl, $CONH_2$; oder

R4 und R5 bilden gemeinsam eine der Gruppen $CH_2$-$CH_2$-$CH_2$-$CH_2$-$CH_2$, $CH_2$-$CH_2$-N($CH_2$-Pheny)-$CH_2$-$CH_2$, $CH_2$-$CH_2$-O-$CH_2$-$CH_2$, $CH_2$-$CH_2$-$CH_2$-$CH_2$;

sowie derer physiologisch verträglichen Salze zur Herstellung eines Medikaments zur Prophylaxe oder Behandlung von Obesitas.

3. Verwendung der Verbindungen der Formel I, gemäß Anspruch 1 oder 2, **dadurch gekennzeichnet, daß** darin bedeuten

Y eine direkte Bindung;

X $CH_2$;

$R_1$ F, Cl, Br, $CF_3$, CN, ($C_1$-$C_6$)-Alkyl, O-($C_1$-$C_6$)-Alkyl, wobei in den Alkylresten ein, mehrere, oder alle Wasserstoff(e) durch Fluor ersetzt sein können;
$SO_2$-$NH_2$, $SO_2$NH($C_1$-$C_6$)-Alkyl, $SO_2$N[($C_1$-$C_6$)-Alkyl]$_2$, $SO_2$-($C_1$-$C_6$)-Alkyl, $SO_2$-($CH_2$)$_n$-Phenyl, wobei n = 0 - 6 sein kann und der Phenylrest bis zu zweifach mit F, Cl, Br, OH, $CF_3$, $NO_2$, CN, $OCF_3$, O-($C_1$-$C_6$)-Alkyl, ($C_1$-$C_6$)-Alkyl, $NH_2$ substituiert sein kann;
Phenyl, -O-Phenyl, wobei der Phenylrest bis zu zweifach substituiert sein kann mit F, Cl, Br, OH, $CF_3$, $NO_2$, CN, $OCF_3$, O-($C_1$-$C_6$)-Alkyl, ($C_1$-$C_6$)-Alkyl;
Pyridyl;

R1' H, F, Cl, Br, $CF_3$, CN, ($C_1$-$C_6$)-Alkyl, O-($C_1$-$C_6$)-Alkyl, wobei in den Alkylresten ein, mehrere, oder

alle Wasserstoff(e) durch Fluor ersetzt sein können;
$SO_2$-$NH_2$, $SO_2NH(C_1$-$C_6)$-Alkyl, $SO_2N[(C_1$-$C_6)$-Alkyl$]_2$, $SO_2$-$(C_1$-$C_6)$-Alkyl, $SO_2$-$(CH_2)_n$-Phenyl, wobei n = 0 - 6 sein kann und der Phenylrest bis zu zweifach mit F, Cl, Br, OH, $CF_3$, $NO_2$, CN, $OCF_3$, O-$(C_1$-$C_6)$-Alkyl, $(C_1$-$C_6)$-Alkyl, $NH_2$ substituiert sein kann;
Phenyl, wobei der Phenylrest bis zu zweifach substituiert sein kann mit F, Cl, Br, OH, $CF_3$, $NO_2$, CN, $OCF_3$, O-$(C_1$-$C_6)$-Alkyl, $(C_1$-$C_6)$-Alkyl;
Pyridyl;

R2 $NH_2$,NHR3,NR4R5;

R3 $(C_1$-$C_6)$Alkyl, CN, CHO, CO-$NH_2$, CH=NH, C(S)-$NH_2$, C(=NH)-NH-Phenyl, wobei der Phenylring bis zu zweifach substituiert sein kann mit F, Cl, Br, OH, $CF_3$, $NO_2$, CN, $OCF_3$, O-$(C_1$-$C_6)$-Aikyl, $(C_1$-$C_6)$ -Alkyl, $NH_2$, NH$(C_1$-$C_6)$-Alkyl, N($(C_1$-$C_6)$-Alkyl$)_2$;

R4 $C_1$-$C_6$-Alkyl;

R4 und R5 $C_1$-$C_6$-Alkyl; oder R5 bilden gemeinsam eine $CH_2$-$CH_2$-$CH_2$-$CH_2$-$CH_2$-Gruppe;

sowie derer physiologisch verträglichen Salze zur Herstellung eines Medikaments zur Prophylaxe oder Behandlung von Obesitas.

**4.** Verwendung der Verbindungen der Formel I gemäß einem oder mehreren der Ansprüche 1 bis 3 in Kombination mit einem oder mehreren anorektischen Wirkstoff(en) zur Herstellung eines Medikaments zur Prophylaxe oder Behandlung der Obesitas.

## Claims

**1.** The use of compounds of the formula I

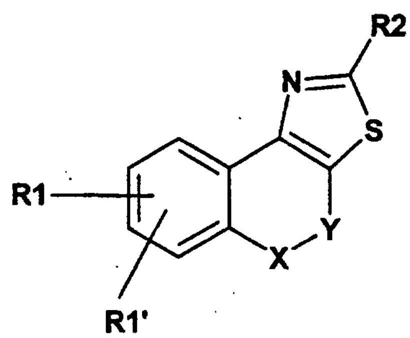

I

in which

Y is a direct linkage, $CH_2$, $CH_2$-$CH_2$;

X is $CH_2$, O, NH, NR6, S;

R1, R1' are, independently of one another H, F, Cl, Br, I, $CF_3$, CN, $NO_2$, COOH, COO$(C_1$-$C_6)$alkyl, $CONH_2$, CONH$(C_1$-$C_6)$alkyl, CON[$(C_1$-$C_6)$alkyl$]_2$, $(C_1$-$C_6)$-alkyl, $(C_2$-$C_6)$-alkenyl, $(C_2$-$C_6)$-alkynyl, O-$(C_1$-$C_6)$-alkyl, where one, more than one or all hydrogen(s) in the alkyl, alkenyl and alkynyl radicals can be replaced by fluorine, or one hydrogen can be replaced by OH, OC(O)$CH_3$, OC(O)H, O-$CH_2$-Ph, NH2, NH-CO-$CH_3$ or N($COOCH_2Ph)_2$;
$SO_2$-$NH_2$, $SO_2NH(C_1$-$C_6)$-alkyl, $SO_2N[(C_1$-$C_6)$-alkyl$)_2$, S-$(C_1$-$C_6)$-alkyl, S-$(CH_2)_n$-phenyl, SO-$(C_1$-$C_6)$-alkyl, SO-$(CH_2)_n$-phenyl, $SO_2$-$(C_1$-$C_6)$-alkyl, $SO_2$-$(CH_2)_n$-phenyl, where n can be 0-6 and the phenyl radical can be substituted up to twice by F, Cl, Br, OH, $CF_3$, $NO_2$, CN, $OCF_3$,

O-($C_1$-$C_6$)-alkyl, ($C_1$-$C_6$)-alkyl, $NH_2$;
$NH_2$, NH-($C_1$-$C_6$-alkyl, N(($C_1$-$C_6$)-alkyl)$_2$, NH($C_1$-$C_7$)-acyl, phenyl, biphenylyl, O-$(CH_2)_n$-phenyl, where n can be 0-6, 1- or 2-naphthyl, 2-, 3- or 4-pyridyl, 2- or 3-furanyl or 2- or 3-thienyl, it being possible for the phenyl, biphenylyl, naphthyl, pyridyl, furanyl or thienyl rings each to be substituted up to 3 times by F, Cl, Br, I, OH, $CF_3$, $NO_2$, CN, $OCF_3$, O-($C_1$-$C_6$)-alkyl, ($C_1$-$C_6$)-alkyl, $NH_2$, NH ($C_1$-$C_6$)-alkyl, N(($C_1$-$C_6$)-alkyl)$_2$, $SO_2$-$CH_3$, COOH, COO-($C_1$-$C_6$)-alkyl, $CONH_2$;
1,2,3-triazol-5-yl, it being possible for the triazol ring to be substituted in position 1, 2 or 3 by methyl or benzyl;
tetrazol-5-yl, it being possible for the tetrazol ring to be substituted in position 1 or 2 by methyl or benzyl;

R2 is $NH_2$, NHR3, NR4R5;

R3 is ($C_1$-$C_6$)alkyl, CN, CHO, CO-$NH_2$, CH=NH, C(S)-$NH_2$, C(=NH)-NH-phenyl, it being possible for the phenyl ring to be substituted up to twice by F, Cl, Br, I, OH, $CF_3$, $NO_2$, CN, $OCF_3$, O-($C_1$-$C_6$)-alkyl, ($C_1$-$C_6$)-alkyl, $NH_2$, NH($C_1$-$C_6$)-alkyl, N(($C_1$-$C_6$)-alkyl)$_2$, $SO_2$-$CH_3$, COOH, COO-($C_1$-$C_6$)-alkyl, $CONH_2$;
phenyl, $CH_2$-phenyl, it being possible for the phenyl ring to be substituted up to 3 times by F, Cl, Br, I, OH, $NO_2$, CN, $OCF_3$, O-($C_2$-$C_6$)-alkyl, ($C_2$-$C_6$)-alkyl, $NH_2$, NH($C_1$-$C_6$)alkyl, N(($C_1$-$C_6$)-alkyl)$_2$, $SO_2$-$CH_3$, COOH, COO-($C_1$-$C_6$)-alkyl, $CONH_2$; biphenylyl, 1- or 2-naphthyl, 4-pyridyl, 2- or 3-furanyl, 2- or 3-thienyl, 5-tetrazolyl, it being possible for the biphenylyl, naphthyl, pyridyl, furanyl or thienyl rings each to be substituted up to twice by F, Cl, Br, I, OH, $CF_3$, $NO_2$, CN, $OCF_3$, O-($C_1$-$C_6$)-alkyl, ($C_1$-$C_6$)-alkyl, $NH_2$, NH($C_1$-$C_6$)-alkyl, N(($C_1$-$C_6$)-alkyl)$_2$, $SO_2$-$CH_3$, COOH, COO-($C_1$-$C_6$)-alkyl, $CONH_2$;
$CH_2$-phenyl, $CH_2$-2-pyridyl or $CH_2$-4-pyridyl, it being possible for the phenyl or pyridyl ring to be substituted once or twice by F, Cl, Br, I, OH, $CF_3$, $NO_2$, CN, $OCF_3$, O-($C_1$-$C_6$)-alkyl, ($C_1$-$C_6$)-alkyl, $NH_2$, NH($C_1$-$C_6$)-alkyl, N(($C_1$-$C_6$)-alkyl)2, $SO_2$-$CH_3$, COOH, COO-($C_1$-$C_6$)-alkyl, $CONH_2$;

R4 is $C_1$-$C_6$-alkyl, $C_3$-$C_6$-cycloalkyl, $C_2$-$C_6$-alkenyl, $C_3$-$C_6$-alkynyl, phenyl, $CH_2$-phenyl, it being possible for the phenyl ring to be substituted once or twice by F, Cl, Br, I, OH, $CF_3$, $NO_2$, CN, $OCF_3$, O-($C_1$-$C_6$)-alkyl, ($C_1$-$C_6$)-alkyl, $NH_2$, NH($C_1$-$C_6$)-alkyl, N(($C_1$-$C_6$)-alkyl)$_2$, $SO_2$-$CH_3$, COOH, COO-($C_1$-$C_6$)-alkyl, $CONH_2$;

R5 is H, $C_1$-$C_6$-alkyl, $C_3$-$C_6$-cycloalkyl, $C_2$-$C_6$-alkenyl, $C_3$-$C_6$-alkynyl, phenyl, $CH_2$-phenyl, it being possible for the phenyl ring to be substituted once or twice by F, Cl, Br, I, OH, $CF_3$, $NO_2$, CN, $OCF_3$, O-($C_1$-$C_6$)-alkyl, ($C_1$-$C_6$)-alkyl, $NH_2$, NH($C_1$-$C_6$)-alkyl, N(($C_1$-$C_6$)-alkyl)$_2$, $SO_2$-$CH_3$, COOH, COO-($C_1$-$C_6$)-alkyl, $CONH_2$; or

R4 and R5 together form one of the groups $CH_2$-$CH_2$-$CH_2$-$CH_2$-$CH_2$, $CH_2$-$CH_2$-N($CH_3$)-$CH_2$-$CH_2$, $CH_2$-$CH_2$-N ($CH_2$-phenyl)-$CH_2$-$CH_2$, $CH_2$-$CH_2$-O-$CH_2$-$CH_2$, $CH_2$-$CH_2$-$CH_2$-$CH_2$;

R6 is ($C_1$-$C_6$)alkyl, acyl, (C=O)-($C_1$-$C_6$)alkyl, (C=O)-($C_3$-$C_6$)-cycloalkyl, phenyl, naphthyl, pyridyl, -$SO_2$-phenyl, $SO_2$-naphthyl;

and their physiologically tolerated salts for producing a medicine for the prophylaxis or treatment of obesity.

**2.** The use of compounds of the formula I as claimed in claim 1, wherein the meanings are as follows

Y a direct linkage, $CH_2$, $CH_2$-$CH_2$;

X $CH_2$, O;

R1 F, Cl, Br, I, $CF_3$, CN, $NO_2$, COOH, COO($C_1$-$C_6$)alkyl, $CONH_2$, CONH($C_1$-$C_6$)alkyl, CON[($C_1$-$C_6$) alkyl]$_2$, ($C_1$-$C_6$)-alkyl, ($C_2$-$C_6$)-alkenyl, ($C_2$-$C_6$)-alkynyl, O-($C_1$-$C_6$)-alkyl, where one, more than one or all hydrogen(s) in the alkyl, alkenyl and alkynyl radicals can be replaced by fluorine, or one hydrogen can be replaced by OH, OC(O)$CH_3$, OC(O)H, O-$CH_2$-Ph, $NH_2$, NH-CO-$CH_3$ or N ($COOCH_2Ph$)$_2$;
$SO_2$-$NH_2$, $SO_2NH$($C_1$-$C_6$)-alkyl, $SO_2N$[($C_1$-$C_6$)-alkyl]$_2$, S-($C_1$-$C_6$)-alkyl, S-$(CH_2)_n$-phenyl,

$SO-(C_1-C_6)$-alkyl, $SO-(CH_2)_n$-phenyl, $SO_2-(C_1-C_6)$-alkyl, $SO_2-(CH_2)_n$-phenyl, where n can be 0-6 and the phenyl radical can be substituted up to twice by F, Cl, Br, OH, $CF_3$, $NO_2$, CN, $OCF_3$, $O-(C_1-C_6)$-alkyl, $(C_1-C_6)$-alkyl, $NH_2$;
phenyl, -O-phenyl, it being possible for the phenyl radical to be substituted up to twice by F, Cl, Br, I, OH, $CF_3$, $NO_2$, CN, $OCF_3$, $O-(C_1-C_6)$-alkyl, $(C_1-C_6)$-alkyl, $NH_2$, $NH(C_1-C_6)$-alkyl, $N((C_1-C_6)\text{-alkyl})_2$, $SO_2-CH_3$, COOH, $COO-(C_1-C_6)$-alkyl, $CONH_2$;

R1' H, F, Cl, Br,I, $CF_3$, CN, $NO_2$, COOH, $COO(C_1-C_6)$alkyl, $CONH_2$, $CONH(C_1-C_6)$alkyl, $CON[(C_1-C_6)\text{alkyl}]_2$, $(C_1-C_6)$-alkyl, $(C_2-C_6)$-alkenyl, $(C_2-C_6)$-alkynyl, $O-(C_1-C_6)$-alkyl, where one, more than one or all hydrogen(s) in the alkyl, alkenyl and alkynyl radicals can be replaced by fluorine, or one hydrogen can be replaced by OH, $OC(O)CH_3$, OC(O)H, $O-CH_2$-Ph, $NH_2$, $NH-CO-CH_3$ or $N(COOCH_2Ph)_2$; .
$SO_2-NH_2$, $SO_2NH(C_1-C_6)$-alkyl, $SO_2N[(C_1-C_6)\text{-alkyl}]2$, $S-(C_1-C_6)$-alkyl, $S-(CH_2)_n$-phenyl, $SO-(C_1-C_6)$-alkyl, $SO-(CH_2)_n$-phenyl, $SO_2-(C_1-C_6)$-alkyl, $SO_2-(CH_2)_n$-phenyl, where n can be 0-6 and the phenyl radical can be substituted up to twice by F, Cl, Br, OH, $CF_3$, $NO_2$, CN, $OCF_3$, $O-(C_1-C_6)$-alkyl, $(C_1-C_6)$-alkyl, $NH_2$;
phenyl, -O-phenyl, it being possible for the phenyl radical to be substituted up to twice by F, Cl, Br, I, OH, $CF_3$, $NO_2$, CN, $OCF_3$, $O-(C_1-C_6)$-alkyl, $(C_1-C_6)$-alkyl, $NH_2$, $NH(C_1-C_6)$-alkyl, $N((C_1-C_6)\text{-alkyl})_2$, $SO_2-CH_3$, COOH, $COO-(C_1-C_6)$alkyl, $CONH_2$;

R2 $NH_2$, NHR3, NR4R5;

R3 $(C_1-C_6)$alkyl, CN, CHO, $CO-NH_2$, CH=NH, $C(S)-NH_2$, C(=NH)-NH-phenyl, it being possible for the phenyl ring to be substituted up to twice by F, Cl, Br, I, OH, $CF_3$, $NO_2$, CN, $OCF_3$, $O-(C_1-C_6)$alkyl, $(C_1-C_6)$-alkyl, $NH_2$, $NH(C_1-C_6)$-alkyl, $N((C_1-C_6)\text{alkyl})_2$, $SO_2-CH_3$, COOH, $COO-(C_1-C_6)$-alkyl, $CONH_2$;
phenyl, $CH_2$-phenyl, it being possible for the phenyl ring to be substituted once to 3 times by F, Cl, Br,I, OH, $NO_2$, CN, $OCF_3$, $O-(C_2-C_6)$-alkyl, $(C_2-C_6)$-alkyl, $NH_2$, $NH(C_1-C_6)$alkyl, $N((C_1-C_6)\text{-alkyl})_2$, $SO_2-CH_3$, COOH, $COO-(C_1-C_6)$-alkyl, $GONH_2$;

R4 $C_1-C_6$-alkyl, $C_3-C_6$-cycloalkyl, $C_2-C_6$-alkenyl, $C_3-C_6$-alkynyl, phenyl, $CH_2$-phenyl, it being possible for the phenyl ring to be substituted once or twice by F, Cl, Br,I, OH, $CF_3$, $NO_2$, CN, $OCF_3$, $O-(C_1-C_6)$-alkyl, $(C_1-C_6)$-alkyl, $NH_2$, $NH(C_1-C_6)$-alkyl, $N((C_1-C_6)\text{-alkyl})_2$, $SO_2-CH_3$, COOH, $COO-(C_1-C_6)$-alkyl, $CONH_2$;

R5 $C_1-C_6$-alkyl, $C_3-C_6$-cycfoalkyl, $C_2-C_6$-alkenyl, $C_3-C_6$-alkynyl, phenyl, $CH_2$-phenyl, it being possible for the phenyl ring to be substituted once or twice by F, Cl, Br,I, OH; $CF_3$, $NO_2$, CN, $OCF_3$, $O-(C_1-C_6)$-alkyl, $(C_1-C_6)$-alkyl, $NH_2$, $NH(C_1-C_6)$-alkyl, $N((C_1-C_6)\text{-alkyl})_2$, $SO_2-CH_3$, COOH, $COO-(C_1-C_6)$-alkyl, $CONH_2$; or

R4 and R5 together form one of the groups $CH_2-CH_2-CH_2$-CH2-$CH_2$, $CH_2-CH_2-N(CH_2\text{-phenyl})-CH_2-CH_2$, $CH_2-CH_2-O-CH_2-CH_2$, $CH_2-CH_2-CH_2-CH_2$;

and their physiologically tolerated salts for producing a medicine for the prophylaxis or treatment of obesity.

**3.** The use of compounds of the formula I as claimed in claim 1 or 2, wherein the meanings are as follows

Y a direct linkage;

X $CH_2$;

R1 F; Cl, Br; $CF_3$, CN, $(C_1-C_6)$-alkyl, $O-(C_1-C_6)$-alkyl, where one, more than one or all hydrogen(s) in the alkyl radicals can be replaced by fluorine;
$SO_2-NH_2$, $SO_2NH(C_1-C_6)$-alkyl, $SO_2N[(C_1-C_6)\text{-alkyl}]_2$, $SO_2-(C_1-C_6)$-alkyl, $SO_2-(CH_2)_n$-phenyl, where n can be 0-6 and the phenyl radical can be substituted up to twice by F, Cl, Br, OH, $CF_3$, $NO_2$, CN, $OCF_3$, $O-(C_1-C_6)$-alkyl, $(C_1-C_6)$-alkyl, $NH_2$; phenyl, -O-phenyl, it being possible for the phenyl radical to be substituted up to twice by F, Cl, Br, OH, $CF_3$, $NO_2$, CN, $OCF_3$, $O-(C_1-C_6)$-alkyl, $(C_1-C_6)$-alkyl;

pyridyl;

R1' H, F, Cl, Br, $CF_3$, CN, $(C_1\text{-}C_6)$-alkyl, O-$(C_1\text{-}C_6)$-alkyl, where one, more than one or all hydrogen(s) in the alkyl radicals can be replaced by fluorine;
$SO_2$-$NH_2$, $SO_2NH(C_1\text{-}C_6)$-alkyl, $SO_2N[(C_1\text{-}C_6)\text{-alkyl}]_2$, $SO_2$-$(C_1\text{-}C_6)$-alkyl, $SO_2$-$(CH_2)_n$-phenyl, where n can be 0-6 and the phenyl radical can be substituted up to twice by F, Cl, Br, OH, $CF_3$, $NO_2$, CN, $OCF_3$, O-$(C_1\text{-}C_6)$-alkyl, $(C_1\text{-}C_6)$-alkyl, $NH_2$;
phenyl, it being possible for the phenyl radical to be . substituted up to twice by F, Cl, Br, OH, $CF_3$, $NO_2$, CN, $OCF_3$, O-$(C_1\text{-}C_6)$alkyl, $(C_1\text{-}C_6)$-alkyl;
pyridyl;

R2 $NH_2$, NHR3, NR4R5;

R3 $(C_1\text{-}C_6)$alkyl, CN, CHO, CO-$NH_2$, CH=NH, C(S)-$NH_2$, C(=NH)-NH-phenyl, it being possible for the phenyl ring to be substituted up to twice by F, Cl, Br, OH, $CF_3$, $NO_2$, CN, $OCF_3$, O-$(C_1\text{-}C_6)$alkyl, $(C_1\text{-}C_6)$-alkyl, $NH_2$, $NH(C_1\text{-}C_6)$-alkyl, $N((C_1\text{-}C_6)\text{-alkyl})_2$;

R4 $C_1$-$C_6$-alkyl;

R5 $C_1$-$C_6$-alkyl; or

R4 and R5 together form a $CH_2$-$CH_2$-$CH_2$-$CH_2$-$CH_2$- group;

and their physiologically tolerated salts for producing a medicine for the prophylaxis or treatment of obesity.

**4.** The use of compounds of the formula I as claimed in one or more of claims 1 to 3 in combination with one or more anorectic active ingredient(s) for producing a medicine for the prophylaxis or treatment of obesity.

## Revendications

**1.** Utilisation des composés de formule I,

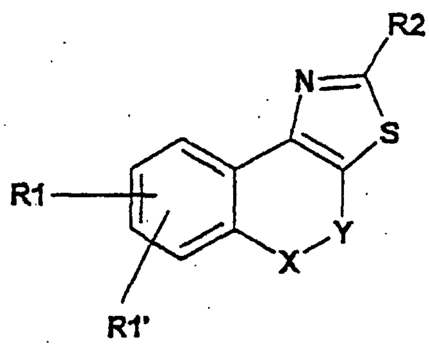

I

où représentent

Y une liaison directe, $CH_2$, $CH_2$-$CH_2$ ;
X $CH_2$, O, NH, $NR_6$, S ;
$R^1$, $R^{1'}$ indépendamment l'un de l'autre un atome d'hydrogène, de F, de Cl, de Br, d'I, un groupe $CF_3$, CN, $NO_2$, COOH, COO-alkyle en $C_1$-$C_6$, $CONH_2$, CONH-alkyle en $C_1$-$C_6$, CON-(alkyle en $C_1$-$C_6$)$_2$, alkyle en $C_1$-$C_6$, alcényle en $C_2$-$C_6$, alcynyle en $C_2$-$C_6$, O-alkyle en $C_1$-$C_6$, dans les restes alkyle, alcényle et alcynyle un, plusieurs ou tous les atomes d'hydrogène peuvent être remplacés par des atomes de fluor, ou un atome d'hydrogène peut être remplacé par des groupes OH, OC(O), $CH_3$, OC(O)H, O-$CH_2$-Ph, $NH_2$, NH-CO-$CH_3$ ou N $(COOCH_2Ph)_2$ ; $SO_2$-$NH_2$, $SO_2NH$-alkyle en $C_1$-$C_6$, $SO_2N$- (alkyle en

$C_1$-$C_6$)$_2$, S-alkyle en $C_1$-$C_6$, S- $(CH_2)_n$-phényle, SO-alkyle en $C_1$-$C_6$, SO-$(CH_2)_n$-phényle, $SO_2$-alkyle en $C_1$-$C_6$, $SO_2$-$(CH_2)_n$-phényle, n = 0-6 et le reste phényle peut être substitué jusqu'à deux fois par des substituants F, Cl, Br, OH, $CF_3$, $NO_2$, CN, $OCF_3$, O-alkyle en $C_1$-$C_6$, alkyle en $C_1$-$C_6$, $NH_2$ ; $NH_2$, NH-alkyle en $C_1$-$C_6$, N-(alkyle en $C_1$-$C_6$)$_2$, NH-acyle en $C_1$-$C_7$, phényle, biphénylyle, O-$(CH_2)_n$-phényle, où n = 0-6, 1- ou 2-naphtyle, 2-, 3- ou 4-pyridyle, 2- ou 3-furanyle ou 2- ou 3-thiényle, où les cycles phényle, biphénylyle, naphtyle, pyridyle, furanyle ou thiényle chacun pouvant être substitués une à trois fois par F, Cl, Br, I, OH, $CF_3$, $NO_2$, CN, $OCF_3$, O-alkyle en $C_1$-$C_6$, alkyle en $C_1$-$C_6$, $NH_2$, NH-alkyle en $C_1$-$C_6$, N(alkyle en $C_1$-$C_6$)$_2$, $SO_2$-$CH_3$, COOH, COO-alkyle en $C_1$-$C_6$, $CONH_2$ ;
1,2,3-triazol-5-yle, le cycle triazole pouvant être substitué en position 1, 2 ou 3 par des substituants méthyle ou benzyle ; tétrazol-5-yle, le cycle tétrazole pouvant être substitué en position 1 ou 2 par des substituants méthyle ou benzyle ;

R2 un groupe $NH_2$, NHR3, NR4R5 ;

R3 un groupe alkyle en $C_1$-$C_6$, CN, CHO, CO-$NH_2$, CH=NH, C(S)-$NH_2$, C(=NH)-NH-phényle, le cycle phényle pouvant être substitué jusqu'à 2 fois par des substituants F, Cl, Br, I, OH, $CF_3$, $NO_2$, CN, $OCF_3$, O-alkyle en $C_1$-$C_6$, alkyle en $C_1$-$C_6$, $NH_2$, NH-alkyle en $C_1$-$C_6$, N- (alkyle en $C_1$-$C_6$) $_2$, $SO_2$-$CH_3$ COOH, COO-alkyle en $C_1$-$C_6$, $CONH_2$ ;
phényle, $CH_2$-phényle, le cycle phényle pouvant être substitué jusqu'à 3 fois par des substituants F, Cl, Br, I, OH, $NO_2$, CN, $OCF_3$,. O-alkyle en $C_2$-$C_6$, alkyle en $C_2$-$C_6$, $NH_2$, NH-alkyle en $C_1$-$C_6$, N-(alkyle en- $C_1$-$C_6$)$_2$, $SO_2$-$CH_3$, COOH, COO-(alkyle en $C_1$-$C_6$), $CONH_2$ ;
biphénylyle napht-1-yle ou -2-yle, pyrid-4-yle, furan-2-yle ou -3-yle, thién-2-yle ou -3-yle, tétrazol-5-yle, les cycles biphénylyle, naphtyle, pyridyle, furanyle ou thiényle chacun pouvant être substitués 1 à 2 fois par des substituants F, Cl, Br, I, OH, $CF_3$, $NO_2$, CN, $OCF_3$, O-alkyle en $C_1$-$C_6$, alkyle en $C_1$-$C_6$, $NH_2$, NH-alkyle en $C_1$-$C_6$, N-(alkyle en $C_1$-$C_6$)$_2$, $SO_2$-$CH_3$, COOH, COO-alkyle en $C_1$-$C_6$, $CONH_2$ ;
$CH_2$-phényle, $CH_2$-pyrid-2-yle ou $CH_2$-pyrid-4-yle, le cycle phényle ou pyridyle pouvant être substitué une à deux fois par des substituants F, Cl, Br, I, OH, $CF_3$, $NO_2$, CN, $OCF_3$, O-alkyle en $C_1$-$C_6$, alkyle en $C_1$-$C_6$, $NH_2$, NH-alkyle en $C_1$-$C_6$, N (alkyle en $C_1$-$C_6$)$_2$, $SO_2$-$CH_3$, COOH, COO-alkyle en $C_1$-$C_6$, $CONH_2$ ;

R4 un groupe alkyle en $C_1$-$C_6$, cycloalkyle en $C_3$-$C_6$, alcényle en $C_2$-$C_6$, alcynyle en $C_3$-$C_6$, phényle, $CH_2$-phényle, le cycle phényle pouvant être substitué une à deux fois par des substituants F, Cl, Br, I, OH, $CF_3$, $NO_2$, CN, $OCF_3$, O-alkyle en $C_1$-$C_6$, alkyle en $C_1$-$C_6$, $NH_2$, NH-alkyle en $C_1$-$C_6$, N(alkyle en $C_1$-$C_6$)$_2$, $SO_2$-$CH_3$, COOH, COO-alkyle en $C_1$-$C_6$, $CONH_2$ ;

R5 un atome d'hydrogène, un groupe alkyle en $C_1$-$C_6$, cycloalkyle en $C_3$-$C_6$,alcényle en $C_2$-$C_6$, alcynyle en $C_3$-$C_6$, phényle, $CH_2$-phényle, le cycle phényle pouvant être substitué une à deux fois par des substituants F, Cl, Br, I, OH, $CF_3$, $NO_2$, CN, $OCF_3$, O-alkyle en $C_1$-$C_6$, alkyle en $C_1$-$C_6$, $NH_2$, NH-alkyle en $C_1$-$C_6$, N(alkyle en $C_1$-$C_6$)$_2$, $SO_2$-$CH_3$, COOH, COO-alkyle en $C_1$-$C_6$, $CONH_2$ ; ou

R4 et R5 forment conjointement un des groupes $CH_2$-$CH_2$-$CH_2$-$CH_2$-$CH_2$, $CH_2$-$CH_2$-N($CH_3$)-$CH_2$-$CH_2$, $CH_2$, -$CH_2$-N ($CH_2$-phényl ) -$CH_2$-$CH_2$, $CH_2$-$CH_2$-O-$CH_2$-$CH_2$, $CH_2$-$CH_2$-$CH_2$-$CH_2$;

R6 alkyle en $C_1$-$C_6$, acyle, (C=O) -alkyle en $C_1$-$C_6$, (C=O)-cycloalkyle en $C_3$-$C_6$, phényle, naphtyle, pyridyle, -$SO_2$-phényle, -$SO_2$-naphtyle ;

ainsi que leurs sels physiologiquement tolérés, pour la préparation d'un médicament pour la prévention ou le traitement de l'obésité.

**2.** Utilisation des composés de formule I, selon la revendication 1, **caractérisée en ce que** représentent

Y une liaison directe, un groupe $CH_2$, $CH_2$-$CH_2$ ;

X un groupe $CH_2$, O ;

R1 un groupe F, Cl, Br, I, $CF_3$, CN, $NO_2$, COOH, COO-alkyle en $C_1$-$C_6$, $CONH_2$, CONH-alkyle en $C_1$-$C_6$, CON(alkyle en $C_1$-$C_6$)$_2$, alkyle en $C_1$-$C_6$, alcényle en $C_2$-$C_6$, alcynyle en $C_2$-$C_6$, O-alkyle en $C_1$-$C_6$, dans les groupes alkyle, alcényle et alcynyle un, plusieurs ou tous les atomes d'hydrogène peuvent être remplacés par des atomes de fluor, ou un atome d'hydrogène peut être remplacé par un groupe OH, OC (O)$CH_3$, OC(O)H, O-$CH_2$-Ph, $NH_2$, NH-CO-$CH_3$ ou N($COOCH_2Ph$)$_2$ ;
$SO_2$-$NH_2$, $SO_2$NH-alkyle en $C_1$-$C_6$, $SO_2$N (alkyle en $C_1$-$C_6$)$_2$, S-alkyle en $C_1$-$C_6$, S-$(CH_2)_n$-phényle, SO-alkyle en $C_1$-$C_6$, SO-$(CH_2)_n$-phényle, $SO_2$-alkyle en $C_1$-$C_6$, $SO_2$-$(CH_2)_n$-phényle, n = 0-6 et le reste phényle peut être substitué jusqu'à deux fois par F, Cl, Br, OH, $CF_3$, $NO_2$, CN, $OCF_3$, O-alkyle en $C_1$-$C_6$, alkyle en $C_1$-$C_6$, $NH_2$ ;
phényle, O-phényle, le reste phényle pouvant être substitué jusqu'à deux fois par des substituants. F, Cl, Br, I, OH, $CF_3$, $NO_2$, CN, $OCF_3$, O-alkyle en $C_1$-$C_6$, alkyle en $C_1$-$C_6$, $NH_2$, NH-alkyle en $C_1$-$C_6$,

N (alkyle en $C_1$-$C_6$)$_2$, $SO_2$-$CH_3$, COOH, COO-alkyle en $C_1$-$C_6$, $CONH_2$ ;

R1' un atome d'hydrogène, de F, de Cl, de Br, d'I, un groupe $CF_3$, CN, $NO_2$, COOH, . COO-alkyle en $C_1$-$C_6$, $CONH_2$, CONH-alkyle en $C_1$-$C_6$, CON-(alkyle, en $C_1$-$C_6$)$_2$, alkyle en $C_1$-$C_6$, alcényle en $C_2$-$C_6$, alcynyle en $C_2$-$C_6$, O-alkyle en $C_1$-$C_6$, dans les groupes alkyle, alcényle et alcynyle un, plusieurs ou tous les atomes d'hydrogène peuvent être remplacés par des atomes de fluor, ou un atome d'hydrogène peut être remplacé par un groupe OH, OC(O)$CH_3$, OC(O)H, O-$CH_2$-Ph, $NH_2$, NH-CO-$CH_3$ ou N($COOCH_2Ph$)$_2$ ;

$SO_2$-$NH_2$, $SO_2$NH-alkyle en $C_1$-$C_6$, $SO_2$N (alkyle en $C_1$-$C_6$)$_2$, S-alkyle en $C_1$-$C_6$, S-($CH_2$)$_n$-phényle, SO-alkyle en $C_1$-$C_6$, SO-($CH_2$)$_n$-phényle, $SO_2$-alkyle en $C_1$-$C_6$, $SO_2$- ($CH_2$)$_n$-phényle, n = 0-6 et le reste phényle peut être substitué jusqu'à deux fois par F, Cl, Br, OH, $CF_3$, $NO_2$, CN, $OCF_3$, O-alkyle en $C_1$-$C_6$, alkyle en $C_1$-$C_6$, $NH_2$ ;

phényle, -O-phényle, le reste phényle pouvant être substitué jusqu'à deux fois par des substituants F, Cl, Br, I, OH, $CF_3$, $NO_2$, CN, $OCF_3$, O-alkyle en $C_1$-$C_6$, alkyle en $C_1$-$C_6$, $NH_2$, NH-alkyle en $C_1$-$C_6$, N(alkyle en $C_1$-$C_6$)$_2$, $SO_2$-$CH_3$, COOH, COO-alkyle en $C_1$-$C_6$, $CONH_2$ ;

R2 un groupe $NH_2$, NHR3, NR4R5 ;

R3 un groupe alkyle en $C_1$-$C_6$, CN, CHO, CO-$NH_2$, CH=NH, C(S)-$NH_2$, C(=NH)-NH-phényle, le cycle phényle pouvant être substitué 1 à 2 fois par F, Cl, Br, I, OH, $CF_3$, $NO_2$, CN, $OCF_3$, O-alkyle en $C_2$-$C_6$, alkyle en $C_2$-$C_6$, $NH_2$, NH-alkyle en $C_1$-$C_6$, N(alkyle en $C_1$-$C_6$)$_2$, $SO_2$-$CH_3$, COOH, COO-alkyle en $C_1$-$C_6$, $CONH_2$ ;

phényle, $CH_2$-phényle, le cycle phényle pouvant être substitué jusqu'à deux fois par F, Cl, Br, I, OH, $NO_2$, CN, $OCF_3$, O-alkyle en $C_1$-$C_6$, alkyle en $C_1$-$C_6$, $NH_2$, NH-alkyle en $C_1$-$C_6$, N(alkyle en $C_1$-$C_6$)$_2$, $SO_2$-$CH_3$, COOH, COO-alkyle en $C_1$-$C_6$, $CONH_2$ ;

R4 un groupe alkyle en $C_1$-$C_6$, cycloalkyle en $C_3$-$C_6$,. alcényle en $C_2$-$C_6$, alcynyle en $C_3$-$C_6$, phényle, $CH_2$-phényle, le cycle phényle pouvant être substitué jusqu'à deux fois par F, Cl, Br, I, OH, $CF_3$, $NO_2$, CN, $OCF_3$, O-alkyle en $C_1$-$C_6$, alkyle en $C_1$-$C_6$, $NH_2$, NH-alkyle en $C_1$-$C_6$, N(alkyle en $C_1$-$C_6$)$_2$, $SO_2$-$CH_3$, COOH, COO-alkyle en $C_1$-$C_6$, $CONH_2$ ;

R5 un groupe alkyle en $C_1$-$C_6$, cycloalkyle en $C_3$-$C_6$, alcényle en $C_2$-$C_6$, alcynyle en $C_3$-$C_6$, phényle, $CH_2$-phényle, le cycle phényle pouvant être substitué une à deux fois par F, Cl, Br, I, OH, $CF_3$, $NO_2$, CN, $OCF_3$, O-alkyle en $C_1$-$C_6$, alkyle en $C_1$-$C_6$, $NH_2$, NH-alkyle en $C_1$-$C_6$, N(alkyle en $C_1$-$C_6$)$_2$, $SO_2$-$CH_3$, COOH, COO-alkyle en $C_1$-$C_6$, $CONH_2$ ou

R4 et R5 forment conjointement un des groupes $CH_2$-$CH_2$-$CH_2$-$CH_2$-$CH_2$, $CH_2$-$CH_2$-N($CH_2$-phényl)-$CH_2$-$CH_2$, $CH_2$-$CH_2$-O-$CH_2$-$CH_2$, $CH_2$-$CH_2$-$CH_2$-$CH_2$ ;

ainsi que leurs sels physiologiquement tolérés, pour la préparation d'un médicament pour la prévention ou le traitement de l'obésité.

3. Utilisation des composés de formule I, selon les revendications 1 ou 2, **caractérisée en ce que** représentent

Y une liaison directe ;

X un groupe $CH_2$, ;

R1 un groupe F, Cl, Br, $CF_3$, CN, alkyle en $C_1$-$C_6$, O-alkyle en $C_1$-$C_6$, dans les restes alkyle un, plusieurs ou tous les atomes d'hydrogène pouvant être remplacés par des atomes de fluor ;

$SO_2$-$NH_2$, $SO_2$NH-alkyle en $C_1$-$C_6$, $SO_2$N (alkyle en $C_1$-$C_6$)$_2$, $SO_2$-alkyle en $C_1$-$C_6$, $SO_2$-($CH_2$)$_n$-phényle, n = 0-6 et le reste phényle pouvant être substitué jusqu'à deux fois par F, Cl, Br, OH, $CF_3$, $NO_2$, CN, $OCF_3$, O-alkyle en $C_1$-$C_6$, alkyle en $C_1$-$C_6$, $NH_2$ ; phényle, -O-phényle, le reste phényle pouvant être substitué jusqu'à deux fois par F, Cl, Br, OH, $CF_3$, $NO_2$, CN, $OCF_3$, O-alkyle en $C_1$-$C_6$, alkyle en $C_1$-$C_6$ ;

pyridyle ;

R1' un atome d'hydrogène, de F, de Cl, de Br, un groupe $CF_3$, CN, alkyle en $C_1$-$C_6$, O-alkyle en $C_1$-$C_6$, dans les restes alkyle un, plusieurs ou tous les atomes d'hydrogène pouvant être remplacés par du fluor ;

$SO_2$-$NH_2$, $SO_2$NH-alkyle en $C_1$-$C_6$, $SO_2$N(alkyle en $C_1$-$C_6$)$_2$, $SO_2$-alkyle en $C_1$-$C_6$, $SO_2$-($CH_2$-)$_n$-phényle, n = 0-6 et le reste phényle pouvant être substitué jusqu'à deux fois par F, Cl, Br, OH, $CF_3$, $NO_2$, CN, $OCF_3$, O-alkyle en $C_1$-$C_6$, alkyle en $C_1$-$C_6$, $NH_2$ ; phényle, le reste phényle pouvant être substitué jusqu'à deux fois par F, Cl, Br, OH, $CF_3$, $NO_2$, CN, $OCF_3$, O-alkyle en $C_1$-$C_6$, alkyle en $C_1$-$C_6$ ;

pyridyle ;

R2 un groupe $NH_2$, NHR3, NR4R5 ;

R3 un groupe alkyle en $C_1$-$C_6$, CN, CHO, CO-$NH_2$, CH=NH, C(S)-$NH_2$, C(=NH)-NH-phényle, le cycle

phényle pouvant être substitué jusqu'à deux fois par F, Cl, Br, OH, $CF_3$, $NO_2$, CN, $OCF_3$, O-alkyle en $C_1$-$C_6$, alkyle en $C_1$-$C_6$, $NH_2$, NH-alkyle en $C_1$-$C_6$, N(alkyle en $C_1$-$C_6$)$_2$ ;

R4 un groupe alkyle en $C_1$-$C_6$ ;

R5 un groupe alkyle en $C_1$-$C_6$ ;ou

R4 et R5 forment conjointement un groupe $CH_2$-$CH_2$-$CH_2$-$CH_2$-$CH_2$ ;

ainsi que leurs sels physiologiquement tolérés, pour la préparation d'un médicament pour la prévention ou le traitement de l'obésité.

**4.** Utilisation des composés de formule I selon une ou plusieurs des revendications 1 à 3 en combinaison avec une ou plusieurs substances actives anorectiques pour la préparation d'un médicament pour la prévention ou le traitement de l'obésité.